# FASCICULE DE BREVET EUROPEEN

(11) **EP 3 568 065 B1**
(45) Date de publication et mention de la délivrance du brevet: **13.03.2024**
(21) Numéro de dépôt: 18702747.9
(22) Date de dépôt: 09.01.2018
(51) Int. Cl.: A61B 5/00, A61B 5/024, A61B 5/026

(54) **CAPTEUR PHYSIOLOGIQUE POUR UNE SPECTROSCOPIE EN PROCHE INFRAROUGE À DIFFÉRENTES PROFONDEURS**
PHYSIOLOGISCHER SENSOR FÜR NAHINFRAROT-SPEKTROSKOPIE AN VERSCHIEDENEN TIEFEN
PHYSIOLOGICAL SENSOR FOR NEAR-INFRARED SPECTROSCOPY AT VARIOUS DEPTHS

(30) Priorité: 10.01.2017 FR 1750207; 03.05.2017 FR 1753888
(43) Date de publication de la demande: 20.11.2019
(73) Titulaire: Braindex S.A.S, 59120 Loos (FR)
(72) Inventeur: CUSSAC, Thierry, 31600 Muret (FR); LUNA ARRIAGA, Antonio, 69100 Villeurbanne (FR)
(74) Mandataire: Germain Maureau
(86) Numéro de dépôt international: PCT/FR2018/050046
(87) Numéro de publication internationale: WO 2018/162808

(56) Documents cités:
- EP-A1- 1 374 778
- EP-A1- 2 092 880
- US-A1- 2012 029 345
- US-A1- 2013 090 541
- US-A1- 2014 275 891
- US-A1- 2014 371 566

## Description

La présente invention se rapporte à un capteur physiologique pour une spectroscopie en proche infrarouge, ainsi qu'à un système de spectroscopie en proche infrarouge comprenant un tel capteur physiologique.

L'invention se situe dans le domaine de la spectroscopie en proche infrarouge pour des applications de diagnostic médical et/ou de surveillance de paramètres physiologiques.

L'invention trouve une application privilégiée dans la surveillance et/ou l'imagerie in vivo de :
- volumes vasculaires ou volumes sanguins, et notamment de volumes vasculaires ou sanguins sous-cutanés, tels qu'un volume sanguin cortical ou tissulaire ou autres ;
- volumes et concentrations d'hémoglobines, et notamment d'oxyhémoglobine (HbO2) et désoxyhémoglobine (Hb) ;
- tests de reperfusion ;
- paramètres hémodynamiques tels que la saturation tissulaire en oxygène (St02), la saturation régionale cérébrale en oxygène (RScO2), le plateau d'autorégulation cérébral ;
- volumes et concentrations de métabolites.

La spectroscopie en proche infrarouge est une technologie classique dans le domaine médical, qui consiste pour l'essentiel à implémenter, d'une part, au moins un émetteur émettant un faisceau de lumière dans le proche infrarouge, avec une longueur comprise entre 650 et 1000 nanomètres, qui traverse les tissus organiques (à la différence de la lumière visible et des ultraviolets) et, d'autre part, au moins un récepteur photosensible qui mesure l'absorption et la dispersion des photons ayant traversé les tissus organiques.

Dans ce spectre spécifique du proche infrarouge, il est possible de détecter les transitions électroniques des chromophores que sont l'oxyhémoglobine et la désoxyhémoglobine. En effet, ces molécules présentes dans le sang voient leur spectre d'absorption changer dans le proche infrarouge en fonction de leur interaction avec l'oxygène, car elles absorbent le spectre du proche infrarouge de manière différentielle en fonction de leur concentration et de leur interaction avec l'oxygène.

Les longueurs d'onde utilisées par l'émetteur sont choisies pour être sensible à ces molécules biologiques (généralement entre 650 et 1000 nanomètres), l'oxyhémoglobine absorbant davantage les longueurs d'onde entre 800 et 1000 nanomètres alors que la désoxyhémoglobine absorbe davantage les longueurs d'onde entre 650 et 800 nanomètres. L'absorption des ondes lumineuses du proche infrarouge par l'oxyhémoglobine et la désoxyhémoglobine sert notamment de base de calcul de la saturation en oxygène en application de la loi de Beer-Lambert, qui stipule que la concentration d'une substance peut être mesurée selon son degré d'absorption de lumière.

Le faisceau de photons émis par un émetteur et reçu par un récepteur prend la forme d'un arc de lumière diffuse qui traverse les tissus organiques, où la profondeur de pénétration du faisceau entre l'émetteur et le récepteur correspond environ au tiers de la distance entre l'émetteur et le récepteur.

Ainsi, la distance entre émetteur et récepteur a un impact considérable sur la profondeur de pénétration du faisceau dans les tissus organiques. Plus un récepteur est éloigné de l'émetteur, plus le faisceau qu'il recueillera aura traversé une grande distance (ou profondeur) dans les tissus organiques, sondant ainsi des couches plus profondes. Une distance entre l'émetteur et le récepteur assez grande est donc nécessaire pour assurer une pénétration suffisante qui permet d'assurer une mesure sur des couches profondes et non sur des couches de surface. Cependant l'augmentation de cette distance émetteur - récepteur aura pour effet une diffusion/absorbtion accrue du signal émis ayant pour conséquence de diminuer la résolution spatiale de la mesure et d'atténuer le signal reçu. En effet, la résolution spatiale de la spectroscopie en proche infrarouge n'excède pas 0,5 millimètres immédiatement sous la peau, et se dégrade la profondeur s'accroissant.

Une application médicale connue de la spectroscopie infrarouge est la mesure de l'activité cérébrale ou de l'oxymétrie cérébrale, en suivant les courbes des concentrations en oxyhémoglobine et désoxyhémoglobine à une profondeur intracrânienne donnée.

Pour cette application, il est connu, notamment des documents WO 2015/109005 et EP 2 434 942, d'employer un capteur physiologique comprenant une bande flexible sur laquelle sont montés un émetteur de faisceaux en proche infrarouge et au moins deux récepteurs photosensibles en proche infrarouge, dont un récepteur proximal séparé d'une distance proximale de l'émetteur et un récepteur distal séparé d'une distance distale de l'émetteur qui est supérieure à la distance proximale. Ainsi, le signal acquis par le récepteur proximal provient d'une source plus superficielle (moins profonde) et le signal acquis par le récepteur distal inclut cette zone superficielle mais aussi une composante plus profonde. En soustrayant le signal proximal du signal distal, il est possible d'obtenir une valeur d'un signal distal exempt des composantes superficielles.

Ainsi, il est prévu d'implémenter plusieurs émetteurs et/ou plusieurs récepteurs, et de procéder à une soustraction spatiale de signaux acquis en spectroscopie en proche infrarouge à différentes profondeurs afin d'augmenter la sélectivité dans certaines applications médicales, dont la mesure de l'activité cérébrale ou de l'oxymétrie cérébrale. Cependant, de tels capteurs physiologiques connus des documents WO 2015/109005 et EP 2 434 942 présentent tout de même une sélectivité limitée.

Cette technique, consistant à mettre en oeuvre une soustraction spatiale de signaux acquis en spectroscopie en proche infrarouge à différentes profondeurs, est également implémentée pour d'autres applications, telles que le suivi des paramètres hémodynamiques ou l'imagerie de volumes vasculaires ou sanguins, ainsi que le décrivent les documents US 2016/0022223, US 2013/0150726 et WO 2015/070162.

Le document US 2016/0022223 pour une application de suivi de paramètres hémodynamiques et le document US 2013/0150726 pour une application de détection d'un volume sanguin de type hématome sous-cranien, proposent chacun de placer des émetteurs et des récepteurs sur une bande support flexible pour pouvoir adapter la bande support sur des parties du corps d'un patient, tels que le crane ou un membre. Cependant, la flexibilité de la bande support apporte une incertitude non maîtrisée sur les positions relatives entre émetteurs et récepteurs, nuisant ainsi à la précision lors des opérations de soustractions spatiales et donc, au final, à une grande imprécision dans les résultats.

Le document WO 2015/070162, dans une application d'imagerie d'un volume sanguin sous-cutanée, propose quant à lui un capteur physiologique comprenant des émetteurs et des récepteurs montés sur un plateau support rigide, qu'il soit plat ou courbe. Cependant, la rigidité du plateau support contribue à limiter la résolution. Par exemple, dans le cas d'un support plat, les émetteurs et les récepteurs sont tous dans un même plan, de sorte que certains d'entre eux ne seront pas plaqués contre la partie concernée du corps du patient, en particulier si cette partie est courbe comme un membre ou un crâne. Ainsi, un tel capteur est fonctionnellement limité dans ses applications par ses propres dimensions quant à la profondeur de tissus explorable, quant à sa résolution et quant à la surface cutanée ou tissulaire sur laquelle l'appliquer. Ce capteur est par exemple inadaptée à une surveillance de l'activité cérébrale ou de l'oxymétrie cérébrale sur des hémisphères frontaux d'un large panel de patients avec des morphologies crâniennes variables, car ce capteur ne peut être fonctionnellement appliqué sur la rotondité de la surface des hémisphères frontaux. En effet, une couche interface d'air apparaîtrait entre la surface cutanée et le capteur, ce qui représenterait une source de diffusion parasitant les faisceaux en proche infrarouge, posant ainsi un problème de modélisation graphique du signal et de la précision des mesures.

L'état de la technique peut également être illustré par l'enseignement du document US 2014/0275891 qui divulgue un capteur physiologique pour une spectroscopie en proche infrarouge à différentes profondeurs, qui comprend trois modules pourvus chacun d'un support (segments) sur lequel est disposé au moins un composant optoélectronique, où chaque support d'un module est relié au support d'au moins un module adjacent au moyen d'une charnière d'articulation.

L'état de la technique peut également être illustré par les documents EP1374778 et US2014/371566 qui divulguent des systèmes de spectroscopie en proche infrarouge mettant en oeuvre respectivement une calotte hémisphérique sur laquelle sont connectées des fibres optiques, et des supports pour des guides optiques reliés par des bandes élastiques.

La présente invention se propose de résoudre les inconvénients précités, en proposant une solution pour pouvoir effectuer une spectroscopie en proche infrarouge à différentes profondeurs avec une résolution accrue et une fiabilité dans les mesures.

Un autre but de l'invention est d'offrir un capteur physiologique qui permette d'effectuer une imagerie tridimensionnelle avec une résolution spatiale améliorée, et ce pour toutes les parties du corps et en particulier les parties courbes telles qu'un organe interne (coeur, foie, ...), un membre (bras, jambe) ou un crâne.

Un autre but de l'invention est d'offrir un capteur physiologique portatif et aisément manipulable par un personnel médical.

Un autre but de l'invention est de permettre une spectroscopie en proche infrarouge à différentes profondeurs, qui compense la variation des chemins optiques dans les tissus traversés du fait de la variation de la rotondité des surfaces examinées, et ce au moyen d'un même capteur physiologique, en vue notamment d'effectuer une imagerie tridimensionnelle avec une résolution spatiale améliorée.

A cet effet, elle propose un système de spectroscopie en proche infrarouge selon la revendication 1.

Ainsi, du fait des articulations d'angulations connues (grâce aux capteurs d'angle) entre les modules élémentaires, les modules élémentaires - et donc les émetteurs et les récepteurs - peuvent être positionnés sur la partie concernée du corps du patient à analyser (par exemple un membre ou un organe interne en environnement aseptisé) de manière précise, connue et maîtrisée, contribuant ainsi à pouvoir adapter la forme du capteur physiologique au plus prêt de la partie concernée du corps du patient, tout en ayant une maîtrise précise des positionnements et des écartements relatifs entre les émetteurs et les récepteurs ; une telle connaissance de ces positionnements et écartements pouvant ensuite être injectés dans un calcul précis et fiable de soustraction spatiale de signaux acquis à différentes profondeurs. Ce calcul pourra également prendre en compte l'angle d'inclinaison de chaque articulation pour compenser la distribution de la lumière dans les tissus biologiques.

Un tel capteur physiologique forme ainsi une nappe optoélectronique ou une nappe de composants optoélectroniques (à savoir les émetteurs et les récepteurs) pouvant se conformer à la surface cutanée de toute partie du corps du patient, grâce aux articulations, ainsi qu'à la morphologie de l'organe sous-jacent, ou de l'organe du patient, et permet une mise en oeuvre d'une spectroscopie dans le proche infrarouge pour détecter in vivo les hémoglobines, ne circulant par nature que dans le réseau vasculaire.

Les articulations entre les modules élémentaires permettent le contact continu ou quasi-continu des composants optoélectroniques avec la surface d'examen, sans création de couche d'air intermédiaire source de diffusion lumineuse.

Par ailleurs, l'angle d'inclinaison de chaque articulation (ou angle d'inclinaison entre deux modules élémentaires) est connu directement et en temps réel grâce aux capteurs d'angle, permettant un traitement précis et efficace pour établir les coordonées spatiales des émetteurs et des récepteurs des différents modules élémentaires.

Un tel capteur physiologique apporte ainsi une réponse au problème de la normalisation des signaux traités, et de la nécessaire compensation de la variation des chemins optiques, induite par la variation de rotondité d'une surface d'examen à une autre, en vue d'une représentation imagée tridimensionnelle, en particulier en employant les capteurs d'angle qui vont effectivement concourrir à atteindre une résolution spatiale accrue et fiabilisée.

Dans un premier mode de réalisation, au moins une articulation entre deux modules élémentaires est une articulation flexible comprenant une bande flexible configurée pour un mouvement relatif entre les modules élémentaires par fléchissement de la bande flexible.

Ainsi, le mouvement relatif entre deux modules élémentaires selon plusieurs angles d'inclinaison est obtenu par un fléchisement (à la manière d'un pliage/dépliage) de la bande flexible formant l'articulation flexible reliant les deux modules élémentaires. La nappe optoélectronique, composée de l'assemblage des modules élémentaires et des articulations, forme donc une nappe au moins en partie souple ou flexible et ajustable à la morphologie de la surface à analyser grâce aux fléchissements des diverses bandes flexibles.

Une articulation avec bande flexible est ainsi dite flexible ou souple, dans le sens où elle est ajustable de manière continue ou non discrète par fléchissement de la bande flexible, offrant ainsi une multiplicité d'angles d'inclinaison.

Selon une possibilité, chaque articulation entre deux modules élémentaires est une articulation flexible comprenant une bande flexible configurée pour un mouvement relatif entre les modules élémentaires par fléchissement de la bande flexible. Autrement dit, toutes les articulations sont des articulations flexibles qui fléchissent pour s'adapter à la morphologie de la surface à analyser.

Selon une caractéristique, chaque module élémentaire comprend un support, rigide ou souple, sur lequel est disposé au moins un composant optoélectronique, et la bande flexible de l'articulation flexible entre deux modules élémentaires est reliée entre les supports desdits deux modules élémentaires.

Dans une réalisation particulière de ce premier mode de réalisation, le capteur physiologique comprend une membrane flexible sur lequel les composants optoélectroniques sont disposés et répartis en ilôts délimitant les modules élémentaires, et où la bande flexible de l'articulation flexible entre deux modules élémentaires est formée d'une partie de la membrane flexible s'étendant entre les ilôts délimitant lesdits deux modules élémentaires.

Selon une autre caractéristique, la membrane flexible comprend au moins une couche en polymère élastomérique dans laquelle les composants optoélectroniques sont au moins partiellement noyés.

Selon une autre caractéristique, pour une articulation flexible comprenant une bande flexible, cette articulation flexible est couplée à un capteur d'angle comprenant un capteur résistif flexible délivrant une donnée de mesure traduisant une variation de résistance en fonction du fléchissement de la bande flexible
Autrement dit, l'articulation flexible est couplée à un capteur résistif flexible ou capteur de flexion. Il est pour cela fait appel à une technologie permettant de mesurer des angles avec précision, basée sur l'exploitation des variations de résistance se produisant lorsqu'un matériau conducteur est courbé ; le capteur résistif flexible se comportant comme un potentiomètre passif dont la résistance varie en fonction du fléchissement.

Selon une première possibilité, le capteur résistif flexible est intégré à la bande flexible, autrement dit la bande flexible qui forme l'articulation flexible entre deux modules élémentaires intègre (intérieurement ou en surface) un tel capteur résistif flexible, en employant un matériau conducteur au moins en partie noyé dans la bande flexible ou fixé en surface sur la bande flexible.

En variante, le capteur résistif flexible est déporté par rapport à la bande flexible, c'est-à-dire que le capteur résistif flexible ne se situe pas sur la bande flexible mais est décalé par rapport à cette bande flexible sur une partie déportée qui va fléchir en même temps que ou avec la bande flexible. Ainsi, le capteur résistif flexible sera apte à mesurer indirectement le fléchissement de la bande flexible en mesurant directement le fléchissement de cette partie déportée.

Avec cette première possibilité, les capteurs résistifs flexibles peuvent ainsi être intégrés à la membrane flexible décrite ci-dessus, notamment en étant déportés sur les côtés de la membrane flexible.

Selon une seconde possibilité, le capteur résistif flexible est intégré à une lame flexible fixée à la bande flexible, notamment de manière détachable, de sorte qu'un fléchissement de la bande flexible conduit à un fléchissement de la lame flexible.

Autrement dit, le capteur résistif flexible comprend un matériau conducteur au moins en partie noyé dans la lame flexible ou fixé en surface sur la lame flexible, et cette lame flexible est rapportée sur la bande flexible formant l'articulation flexible entre deux modules élémentaires. La lame flexible étant attachée à la bande flexible, celle-ci fléchit avec la bande flexible de sorte que la mesure effectuée par le capteur résistif flexible intégré à la lame flexible traduit bien le fléchissement de la bande flexible et donc délivre une mesure de l'angle d'inclinaison entre les deux modules élémentaires.

Ainsi, la fonction de capteur d'angle est assuré sur la lame flexible tandis que la fonction d'articulation est assuré par la bande flexible, dissociant ainsi la partie optoélectronique (les composants optoélectroniques reliés par les articulations flexibles) de la partie mesure d'angle (les capteurs d'angle sur les lames flexibles).

Dans une réalisation particulière, le capteur physiologique comprend un film flexible sur lequel des capteurs résistifs flexibles sont disposés, où ce film flexible est superposé et fixé sur la membrane flexible, et où ce film flexible définit des lames flexibles superposées ou déportées par rapport aux les bandes flexibles des articulations flexibles entre les modules élémentaires, les capteurs résistifs flexibles étant prévus sur ces lames flexibles.

Autrement dit, les capteurs résistifs flexibles sont prévus sur le film flexible qui est lui-même rapporté sur la membrane flexible recevant les composants optoélectroniques, dissociant ainsi la partie optoélectronique (la membrane flexible) de la partie mesure d'angle (le film flexible).

Selon une autre caractéristique, le film flexible comprend au moins une couche en polymère élastomérique dans laquelle les capteurs résistifs flexibles sont au moins partiellement noyés.

Dans un second mode de réalisation, au moins une articulation entre deux modules élémentaires est une articulation semi-rigide configurée pour un mouvement relatif entre les modules élémentaires selon plusieurs angles d'inclinaison prédéfinis discrets, où ladite articulation semi-rigide est configurée pour maintenir une inclinaison stable entre deux modules élémentaires dans chacun desdits angles d'inclinaison, et pour basculer entre deux angles d'inclinaison distincts sous l'effet d'un effort appliqué sur l'un au moins des deux modules élémentaires concernés.

Une telle articulation est semi-rigide, dans le sens où, à la différence de la bande flexible précédement décrite, cette articulation semi-rigide n'est pas ajustable de manière continue et sans stabilité, mais plutôt de manière discrète avec plusieurs angles d'inclinaison prédéfinis où, pour chaque angle d'inclinaison, l'articulation conserve cet angle d'inclinaisons de manière stable.

Selon une possibilité, une articulation semi-rigide est munie de crans de réglage de l'angle d'inclinaison entre les deux modules élémentaires selon plusieurs valeurs prédéfinies.

Dans une réalisation particulière, une articulation semi-rigide est couplée à un capteur d'angle comprenant des contacteurs électriques, où chaque contacteur électrique est couplé à un cran de réglage.

Autrement dit, chaque cran de réglage est couplé à un contacteur, l'ensemble de ces contacteurs formant le capteur d'angle propre à établir la mesure de l'angle d'inclinaison, chaque cran de réglage étant en effet associé à un angle d'inclinaison.

Selon une autre possibilité, une articulation semi-rigide est couplée à un capteur d'angle réalisé sous la forme d'un capteur résistif flexible rapporté sur l'articulation et comprenant une lame flexible qui fléchit avec l'articulation, ledit capteur résistif flexible délivrant une donnée de mesure traduisant une variation de résistance en fonction du fléchissement de la lame flexible.

Selon une autre possibilité, chaque articulation (que ce soit une articulation flexible ou une articulation semi-rigie) est ajustable entre - 30 et + 30 degrés pour l'angle d'inclinaison entre les deux modules élémentaires.

Selon une autre possibiilité, chaque articulation semi-rigide est ajustable selon plusieurs angles d'inclinaison prédéfinis séparés deux à deux d'un pas angulaire constant, notamment un pas angulaire compris entre 5 et 15 degrés.

A titre d'exemples non limitatifs, chaque articulation semi-rigide peut présenter les angles d'inclinaison suivants, en degrés, [0, 5, 10, 15, 20, 25, 30] ou [0, 10, 20, 30] ou [0, 15, 30].

Dans une réalisation particulière, chaque articulation semi-rigide comprend plusieurs billes coopérant avec un élément élastique de poussée, lesdites billes étant mobiles dans un rail courbe d'une courbure prédéfinie et coulissant à l'intérieur d'un guide de même courbure, et comprend en outre sur le guide un curseur apte à écraser les billes selon le coulissement du rail au sein du guide, où les supports liés par l'articulation sont respectivement fixés à une extrémité du rail et à une extrémité du guide.

Ainsi, une telle articulation semi-rigide comprend un rail coulissant dans un guide, avec les billes et l'élément élastique de poussée sur le rail. Le rail et le guide en butée l'un dans l'autre représentent un secteur d'angle correspondant sensiblement à la moitié du secteur d'angle d'inclinaison possible.

Dans ce mode de réalisation, le curseur du guide peut établir le contact électrique avec la bille écrasée correspondant au coulissement du rail dans le guide, permettant ainsi d'établir l'angle d'inclinaison.

En variante, chaque articulation semi-rigide comprend deux demi-faces de charnière pleines, d'environ la moitié du secteur d'angle d'inclinaison maximum souhaité et ayant des reliefs complémentaires qui s'encastrent lorsque lesdites demi-faces de charnière sont plaquées l'une contre l'autre. Ces demi-faces de charnière sont aptes à glisser l'une contre l'autre de façon contrôlée par application d'un effort adapté, de sorte que ces demi-faces de charnière s'écartent légèrement pour passer d'un cran de réglage au cran de réglage suivant. Un contacteur peut permettre de connaître la position du cran de réglage.

Selon une caractéristique, chaque module élémentaire comprend au moins un composant optoélectronique choisi parmi les émetteurs et les récepteurs et disposé selon au moins l'une des dispositions suivantes :
- au moins un émetteur et au moins un récepteur ;
- au moins deux émetteurs de signaux dans des longueurs distinctes dans le proche infrarouge ;
- au moins un émetteur disposé au centre du module élémentaire et au moins un récepteur disposé en périphérie du module élémentaire ;
- au moins un émetteur disposé au centre du module élémentaire et plusieurs récepteurs disposés en périphérie autour dudit émetteur ;
- un ou plusieurs émetteurs et aucun récepteur ;
- un ou plusieurs récepteurs et aucun émetteur.

Selon une autre caractéristique, dans la configuration plane de la nappe optoélectronique, les modules élémentaires sont répartis en plusieurs lignes et en plusieurs colonnes orthogonales aux lignes.

Une telle répartition en lignes et colonnes est avantageuse pour le traitement selon une méthode de soustraction spatiale.

Dans un mode de réalisation particulier, chaque module élémentaire comprend un support, rigide ou souple, sur lequel est disposé au moins un composant optoélectronique, et chaque support comprend deux bords longitudinaux opposés et parallèles, et deux bords transversaux opposés et parallèles, et chaque articulation relie soit deux bords transversaux contigus de deux supports respectifs adjacents, soit deux bords longitudinaux contigus de deux supports respectifs adjacents.

Selon une caractéristique, dans la configuration plane de la nappe optoélectronique, chaque articulation entre deux modules élémentaires est une articulation pivotante autour d'un axe d'articulation s'étendant dans le plan principal ; ce pivotement étant obtenu par fléchissement (ou pliage) de la bande flexible de l'articulation flexible, ou bien est otenu par pivotement entre deux angles d'inclinaison discrets de l'articulation semi-rigide.

L'invention se rapporte donc à un système de spectroscopie en proche infrarouge, comprenant un capteur physiologique tel que décrit ci-dessus, et comprenant en outre une unité de contrôle configurée pour établir les coordonnées spatiales de chaque émetteur et de chaque récepteur en fonction des angles d'inclinaison des articulations, où ladite unité de contrôle est reliée, d'une part, aux émetteurs du capteur physiologique pour activer lesdits émetteurs de manière individuelle et non concomitante selon une séquence prédéfinie et, d'autre part, aux récepteurs du capteur physiologique pour une récupération et un traitement tridimensionnel des mesures issues des récepteurs durant ladite séquence en fonction des coordonnées spatiales des émetteurs et des récepteurs, et dans lequel l'unité de contrôle est reliée à chaque capteur d'angle pour établir les coordonnées spatiales de chaque émetteur et de chaque récepteur en fonction des données de mesure issues desdits capteurs d'angle.

Un tel système de spectroscopie est particulièrement adapté pour toutes les parties du corps, dont les membres et les organes internes (tel que le coeur) grâce à la conformation du capteur physiologique, et grâce à l'unité de contrôle qui permet d'activer les émetteurs selon une séquence prédéfinie et de récupérer les mesures issues des récepteurs. A chaque activation d'un émetteur, l'unité de contrôle prendra en considération certains émetteurs (comme par exemple des paires d'émetteurs alignés avec l'émetteur activé) et ne tiendra pas compte de certains autres émetteurs (en particulier ceux situés au-delà d'une distance seuil prédéfinie). Ainsi, il sera possible de quadriller une zone d'examen à différentes profondeurs, avec une résolution particulièrement fine. Le traitement des mesures issues des récepteurs est un traitement dit tridimensionnel dans la mesure où il procure un résultat en profondeur de la partie du corps analysée.

A titre d'exemples de traitement tridimensionnel, l'unité de contrôle est configurée pour un traitement tridimensionnel des mesures issues des récepteurs durant la séquence selon une méthode de soustraction spatiale, ou selon une méthode de tomographie avec établissement de cartes de contrastes, ou encore selon une méthode de reconstruction à nuage de points.

La méthode de soustraction spatiale met en oeuvre des soustractions entre des mesures issues de récepteurs sélectionnés.

La méthode de tomographie est obtenue par exemple par l'établissement de cartes de contrastes aux différentes longueurs d'onde.

La méthode de reconstruction à nuage de points met quant à elle en oeuvre une considération de toutes les valeurs d'absorption par chaque récepteur à chaque longueur d'onde comme constituant un nuage de points. Ensuite, un algorithme de reconstruction (comme par exemple un algorithme de classification par « machine learning ») permettra d'identifier une cohérence en lien avec les différents volumes d'absorption homogène, afin de permettre une représentation graphique, autrement dit cet algorithme de reconstruction sera appliqué sur l'ensemble de points acquis au cours du temps de manière à mieux distinguer les zones caractérisés par la technique de mesure. Un coefficient de certitude pourrait être attribué aux points fortement répétitifs, ce qui permettrait d'enlever des ambiguïtés sur la mesure lors d'une acquisition bruité par le mouvement du patient par exemple.

Selon une caractéristique, l'unité de contrôle est configurée pour traiter selon un traitement tridimensionnel (eg. selon une méthode de soustraction spatiale, une méthode de tomographie, ou une méthode de reconstruction à nuage de points), à chaque activation d'un émetteur, uniquement des mesures issues de récepteurs sélectionnés en fonction d'une localisation relative vis-à-vis dudit émetteur activé établie à partir de leurs coordonnées spatiales.

Avantageusement, dans la configuration plane de la nappe optoélectronique, les modules élémentaires sont répartis en plusieurs lignes et en plusieurs colonnes orthogonales aux lignes, et l'unité de contrôle est configurée pour traiter selon une méthode de soustraction spatiale, à chaque activation d'un émetteur, les mesures issues de récepteurs disposés dans la même ligne et dans la même colonne que ledit émetteur.

En complément, il est envisageable que l'unité de contrôle soit configurée pour traiter selon une méthode de soustraction spatiale, à chaque activation d'un émetteur, les mesures issues de récepteurs également disposés dans une ligne et dans une colonne différentes de celles dudit émetteur.

L'invention se rapporte aussi à un procédé de spectroscopie en proche infrarouge mettant en oeuvre un système de spectroscopie selon l'invention, un tel procédé de spectroscopie comprenant les étapes suivantes :
- collecte des données de mesure issues des capteurs d'angle couplés à chaque articulation ;
- établissement des coordonnées spatiales de chaque émetteur et de chaque récepteur en fonction des données de mesure issues desdits capteurs d'angle (étant rappelé que ces données de mesure dépendents ou sont représentatives des angles d'inclinaison des articulations) ;
- activation des émetteurs de manière individuelle et non concomitante selon une séquence prédéfinie ;
- récupération et traitement tridimensionnel (eg. selon une méthode de soustraction spatiale, une méthode de tomographie, ou une méthode de reconstruction à nuage de points) des mesures issues des récepteurs durant ladite séquence en fonction des coordonnées spatiales des émetteurs et des récepteurs.

Comme décrit précédemment, il est envisageable que le traitement tridimensionnel des mesures issues des récepteurs durant la séquence mette en oeuvre une méthode de soustraction spatiale, ou une une méthode de tomographie avec établissement de cartes de contrastes, ou une méthode de reconstruction à nuage de points.

Dans un mode de réalisation particulier, l'étape de récupération et traitement est suivie d'une étape de construction d'une image tridimensionnelle représentant les strates tissulaires sous-jacentes, et notamment un volume vasculaire ou sanguin dans un volume d'analyse, ledit volume vasculaire ou sanguin étant composé de voxels construits à partir du traitement tridimensionnel des mesures issues des récepteurs durant la séquence.

Il est à noter que l'étape d'activation et l'étape de récupération et traitement peuvent être répétées à intervalles de temps successifs, afin de permettre un traitement dynamique dans le temps pour suivre une évolution en temps réel.

Avantageusement, l'étape d'établissement des coordonnées spatiales se traduit par la génération d'une empreinte optoélectronique tridimensionnelle et virtuelle du capteur physiologique en situation.

D'autres caractéristiques et avantages de la présente invention apparaîtront à la lecture de la description détaillée ci-après, d'exemples de mise en oeuvre non limitatifs, faite en référence aux figures annexées dans lesquelles :
- la figure 1a est une vue schématique de face d'un premier capteur physiologique conforme à l'invention ;
- la figure 1b est une vue schématique de face d'un module élémentaire du premier capteur physiologique de la figure 1a ;
- la figure 2a est une vue schématique de face d'un deuxième capteur physiologique conforme à l'invention ;
- la figure 2b est une vue schématique de face d'un module élémentaire du deuxième capteur physiologique de la figure 2a ;
- la figure 3a est une vue schématique de face d'un troisième capteur physiologique conforme à l'invention ;
- la figure 3b est une vue schématique de face d'un module élémentaire du troisième capteur physiologique de la figure 3a ;
- la figure 4 est une vue schématique de face de six modules élémentaires du premier capteur physiologique de la figure 1a, illustrant les faisceaux en proche infrarouge qui sont pris en considération lors de l'activation d'un émetteur ;
- la figure 5a est une vue schématique de côté d'un capteur physiologique conforme à l'invention en conformation plane, avec l'illustration de faisceaux en proche infrarouge ;
- la figure 5b est une vue schématique de côté du capteur physiologique de la figure 5a en conformation incurvée, avec l'illustration de faisceaux en proche infrarouge ;
- la figure 6 est une illustration schématique d'une soustraction spatiale de faisceaux pour la construction d'un voxel sous un capteur physiologique conforme à l'invention ;
- les figures 7a et 7b sont des vues schématiques du voxel de la figure 6 dans le cas d'une imagerie d'un volume sanguin de type hématome (figure 7a) ou de type artère (figure 7b) ;
- la figure 8a est une vue schématique de face d'un quatrième capteur physiologique conforme à l'invention ;
- la figure 8b est une vue schématique de face d'un module élémentaire du quatrième capteur physiologique de la figure 8a ;
- la figure 9a est une vue schématique en perspective d'un cinquième capteur physiologique conforme à l'invention ;
- la figure 9b est une vue zoomée sur la zone IX de la figure 9a ;
- la figure 9c est une vue schématique en perspective d'un module élémentaire du cinquième capteur physiologique de la figure 9a ;
- la figure 10 est une vue schématique en perspective d'un sixième capteur physiologique conforme à l'invention.

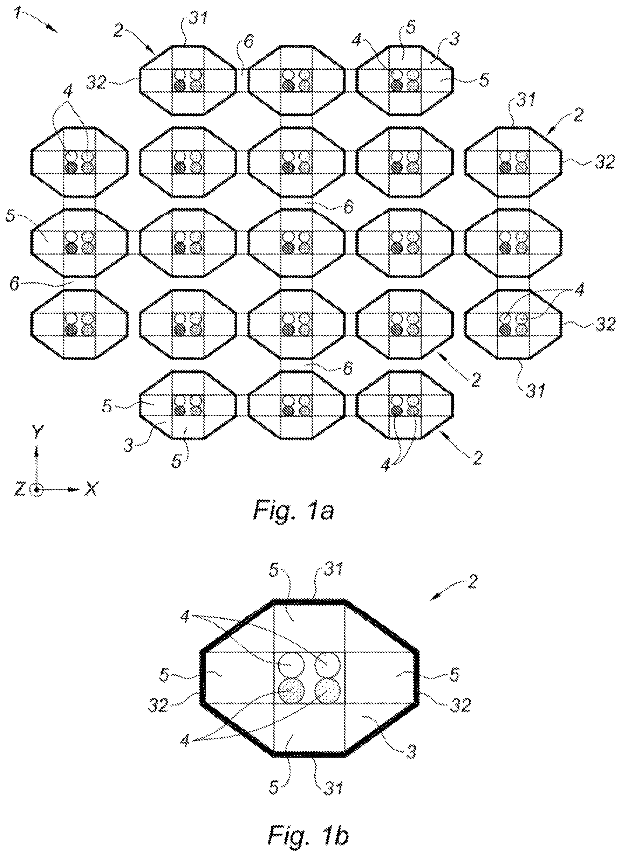

Les figures 1a, 1b, 2a, 2b, 3a, 3b, 8a, 8b, 9a, 9b, 9c et 10 représentent schématiquement six modes de réalisation d'un capteur physiologique 1 conforme à l'invention, pour une spectroscopie en proche infrarouge à différentes profondeurs. Pour la suite de la description, les mêmes références numériques seront employés pour décrire les mêmes éléments structurels ou fonctionnels associés au capteur physiologique 1.

De manière générale, le capteur physiologique 1 comprend une pluralité de modules élémentaires 2 (ou modules optoélectroniques élémentaires) connectés entre eux pour former une nappe optoélectronique flexible ou articulée.

Un capteur physiologique 1 peut comprendre au moins dix modules élémentaires 2, et notamment au moins vingt modules élémentaires 2 ; le nombre de modules élémentaires 2 étant lié à la surface de la zone à analyser.

Chaque module élémentaire 2 est pourvu d'un support 3 (rigide ou souple), réalisé au moins partiellement dans un matériau rigide ou souple, tel qu'un matériau plastique, polymérique, élastomère, composite ou métallique.

Ce module élémentaire 2 comprend en outre au moins un composant optoélectronique de type émetteur 4 de faisceaux en proche infrarouge et/ou de type récepteur 5 photosensibles en proche infrarouge, autrement dit le module élémentaire 2 comprend :
- au moins un émetteur 4 de faisceaux en proche infrarouge ; ou
- au moins un récepteur 5 photosensibles en proche infrarouge ; ou
- au moins un émetteur 4 de faisceaux en proche infrarouge et au moins un récepteur 5 photosensibles en proche infrarouge.

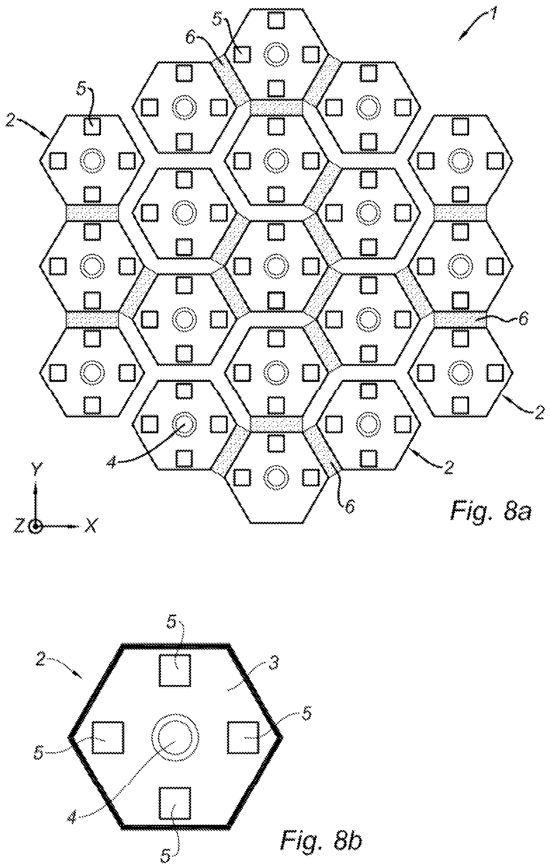

Le support 3 peut présenter une forme générale polygonale, et notamment une forme triangulaire, carrée, rectangulaire, hexagonale, octogonale, etc. Dans les exemples illustrés sur les figures 1a à 3b, le support 3 présente une forme générale octogonale. Dans l'exemple des figures 8a et 8b, le support 3 présente une forme générale hexagonale.

Chaque support 3 d'un module élémentaire est relié au support 3 d'au moins un module élémentaire adjacent au moyen d'une articulation 6 entre les deux supports 3.

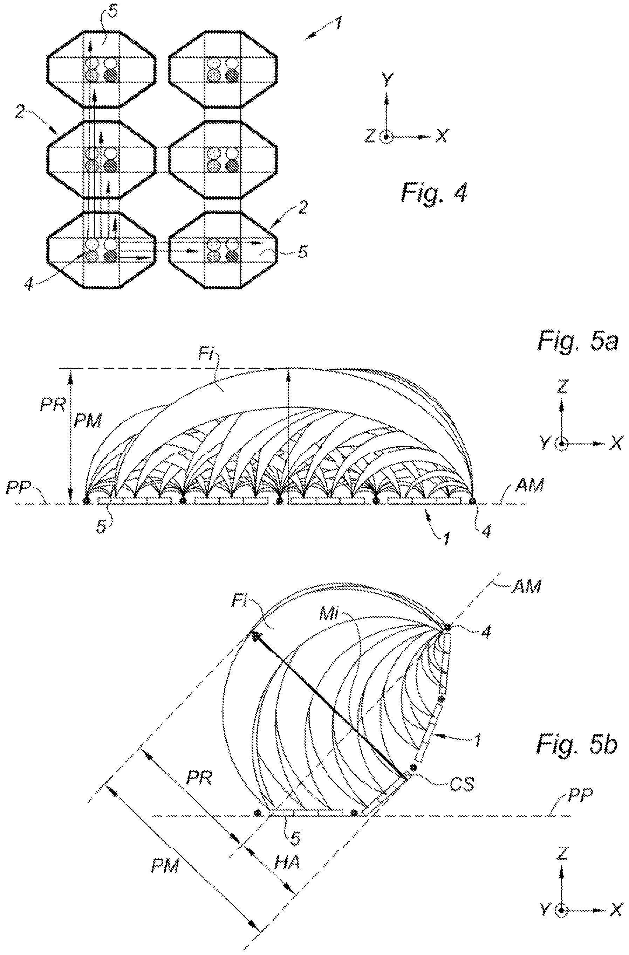

Ainsi, les supports 3 des modules élémentaires 2 sont assemblés ou joints par les articulations 6 de manière à ce que le capteur physiologique 1 forme une nappe optoélectronique configurable entre :
- une configuration plane (illustrée sur la figure 5a) selon un plan principal PP dans laquelle chaque articulation 6 présente un angle d'inclinaison nul vis-à-vis de ce plan principal PP ; et
- une multiplicité de conformations incurvées (dont une est illustrée sur la figure 5b) dans laquelle l'une au moins des articulations 6 présente un angle d'inclinaison non nul vis-à-vis de ce plan principal PP.

Dans la configuration plane du capteur physiologique 1 (ou de la nappe optoélectronique articulée), chaque articulation 6 est une articulation pivotante autour d'un axe d'articulation s'étendant dans le plan principal PP.

Sur les figures 1a, 2a et 3a, le plan principal PP correspond au plan (X, Y) défini par un axe longitudinal X et un axe transversal Y orthogonal à l'axe longitudinal X ; il est également représenté un axe vertical Z orthogonal au plan principal PP ou (X, Y).

Dans un premier mode de réalisation, chaque articulation 6 est une articulation flexible comprenant une bande flexible joignant les deux supports 3 concernés, où la bande flexible est adaptée pour fléchir et ainsi pour permettre un ajustement continu de l'angle d'inclinaison entre les deux supports 3 ; cet ajustement étant continu dans la sens où l'angle d'inclinaison peut varier de manière continue, c'est-à-dire non discrète, par fléchissement (oui pliage) de la bande flexible. Une telle bande flexible est réalisée au moins partiellement dans un matériau flexible et notamment un élastomère. Ainsi, cette articulation 6 flexible est pivotante par fléchissement ou pliage de la bande flexible, qui fléchit autour d'une ligne de fléchissement (ou ligne de pliage) qui forme l'axe d'articulation.

Ainsi, dans ce premier mode de réalisation, le capteur physiologique 1 épouse naturellement la partie du corps du patient. Autrement dit, ce capteur physiologique 1 s'appuie sur une nappe (ou matrice) flexible ou souple de modules élémentaires 2 pouvant se conformer à la partie du corps du patient à analyser, et notamment à la surface cutanée ainsi qu'à la morphologie de l'organe sous-jacent.

Dans le cadre de ce premier mode de réalisation, il est envisageable de prévoir une réalisation dans laquelle tout ou partie des composants optoélectroniques 4, 5 sont intégrés à une même membrane flexible 300 qui formera un support commun à l'ensemble des composants optoélectroniques 4, 5.

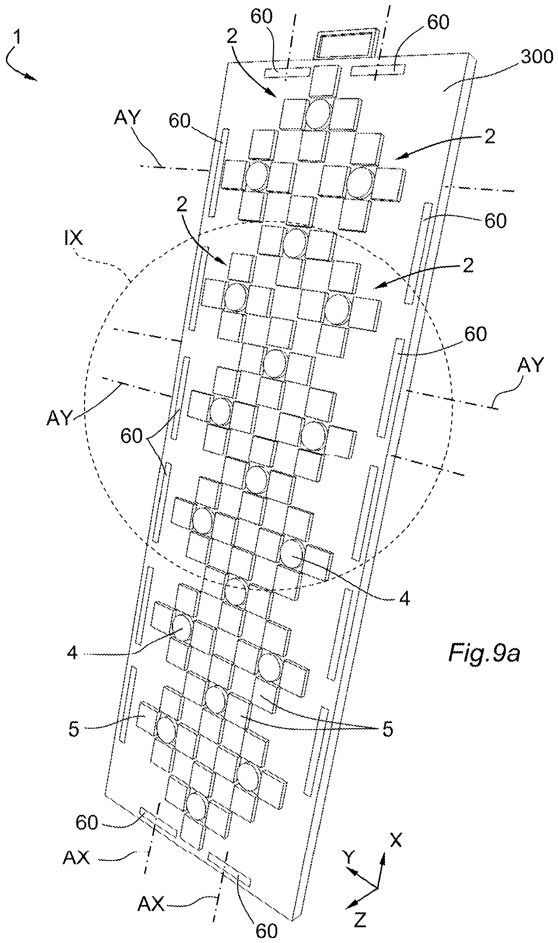

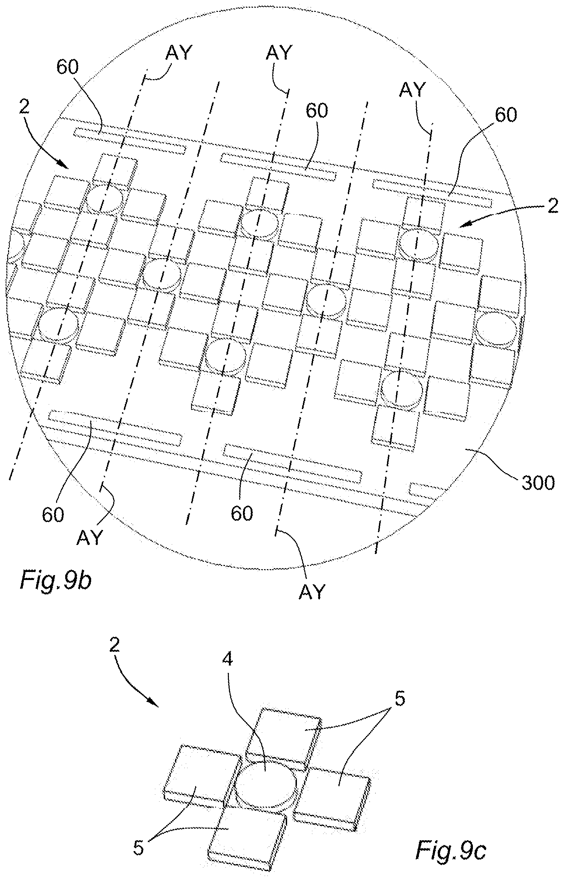

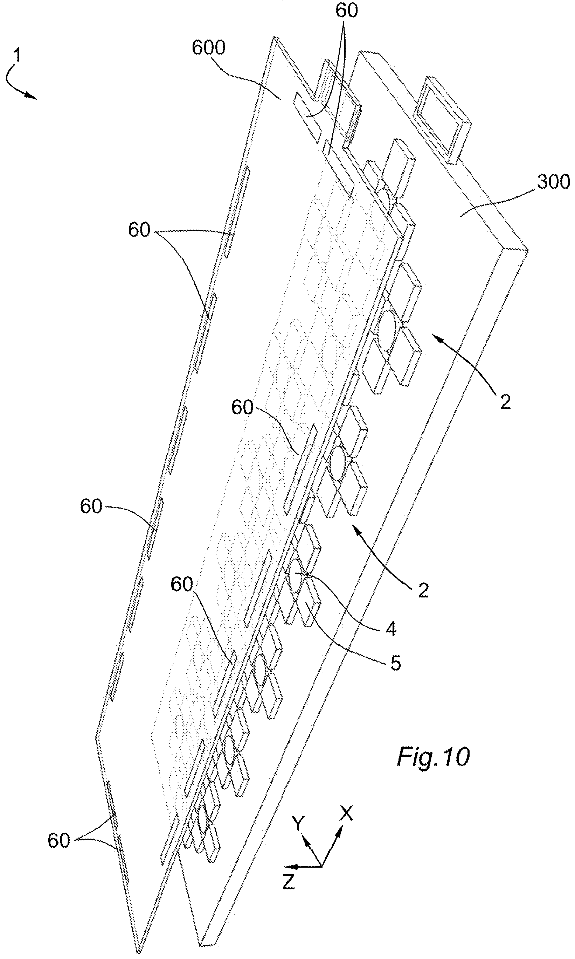

Une telle réalisation avec membrane flexible 300 est illustrée à titre d'exemples sur les figures 9a à 9c avec le cinquième capteur physiologique 1 et sur la figure 10 avec le sixième capteur physiologique 1. Dans cette réalisation, le capteur physiologique 1 comprend une membrane flexible 300 sur lequel les composants optoélectroniques 4, 5 sont disposés et répartis en ilôts délimitant les modules élémentaires 2, où la membrane flexible 300 peut comprendre au moins une couche en polymère élastomérique dans laquelle les composants optoélectroniques 4, 5 sont au moins partiellement noyés. Dans ce cas, les bandes flexibles des articulations flexibles correspondent aux parties de la membrane flexible 300 situés entre les ilôts délimitant les modules élémentaires 2, et les supports des modules élémentaires 2 correspondent aux ilôts ou parties de la membrane flexible 300 sur laquelle sont disposés le ou les composants optoélectroniques 4, 5.

Dans un second mode de réalisation, chaque articulation 6 est une articulation semi-rigide qui autorise plusieurs angles d'inclinaison, de sorte que l'angle d'inclinaison entre deux supports 3 est ajustable de manière discrète par échelons ou pas d'angles d'inclinaison déterminés. Ainsi, une articulation 6 semi-rigide est configurée pour maintenir une inclinaison stable entre deux supports 3 selon les angles d'inclinaison discrets offerts par cette articulation 6 semi-rigide. En outre, une articulation 6 semi-rigide peut basculer entre deux angles d'inclinaison distincts sous l'effet d'un effort appliqué sur l'un au moins des deux supports 3 concernés, en particulier lorsque le capteur physiologique 1 est plaqué ou appliqué sur la partie du corps du patient à analyser.

Ainsi dans ce second mode de réalisation, le capteur physiologique 1 épouse naturellement la partie du corps du patient, en notant tout de même que les angles d'inclinaison entre les supports 3 correspondront aux angles d'inclinaison discrets. Autrement dit, ce capteur physiologique 1 s'appuie sur une nappe (ou matrice) articulée de modules élémentaires 2 pouvant se conformer à la partie du corps du patient à analyser, et notamment à la surface cutanée ainsi qu'à la morphologie de l'organe sous-jacent.

Il est à noter que chaque module élémentaire 2 est relié à au moins un module élémentaire 2 adjacent (ou voisin) via une articulation 6. Un ou plusieurs modules élémentaires 2 peuvent être reliés à quatre modules élémentaires 2 adjacents, en particulier pour les modules élémentaires 2 disposés en partie centrale du capteur physiologique 1. D'autres modules élémentaires 2 peuvent être reliés à un, deux ou trois modules élémentaires 2 adjacents, en particulier pour les modules élémentaires 2 disposés en bordure du capteur physiologique 1. La disposition des articulations 6 dépendra notamment des applications visées, et en particulier de la forme et de l'étendue de la zone à analyser.

Comme visible sur les figures 1a, 2a, 3a, 8a, 9a et 10, dans la configuration plane du capteur physiologique 1 (ou de la nappe optoélectronique), les modules élémentaires 2 sont répartis en :
- plusieurs lignes parallèles à l'axe longitudinal X ; et en
- plusieurs colonnes parallèles à l'axe transversal Y et donc orthogonales aux lignes.

Dans les exemples des figures 1a à 3b, chaque support 3 comprend :
- deux bords longitudinaux 31 opposés et parallèles à l'axe longitudinal X ; et
- deux bords transversaux 32 opposés et parallèles à l'axe transversal Y.

Les formes, dimensionnements et agencements des supports 3 peuvent varier selon les applications visées, et notamment selon les parties du corps ou les organes concernés (coeur, bras, crâne, jambe, etc.), et aussi les types de patients (adulte, enfant, bébé, homme, femme). Pour rappel, dans les réalisations des figures 9a à 9c et 10, le support est commun aux composants optoélectroniques 4, 5 et est réalisée sous la forme d'une membrane fexible 300.

Chaque articulation 6 relie :
- soit deux bords transversaux 32 contigus de deux supports 3 respectifs adjacents, avec un axe d'articulation parallèle à l'axe transversal Y ;
- soit deux bords longitudinaux 31 contigus de deux supports 3 respectifs adjacents, avec un axe d'articulation parallèle à l'axe longitudinal X.

Dans les exemples des figures 8a et 8b, chaque support 3 comprend six bords, et chaque articulation 6 relie deux bords contigus de deux supports 3 respectifs adjacents.

Les articulations 6 entre les modules élémentaires 2 permet ainsi le contact continu (ou quasi continu) des émetteurs 4 et récepteurs 5 avec la surface d'examen, sans création de couche d'air intermédiaire source de diffusion lumineuse.

Dans le premier mode de réalisation décrit ci-dessus où les articulations 6 sont des articulations flexibles, chaque articulation 6 flexible forme une charnière flexible qui contribue à épouser naturellement la forme ou contour de la partie du corps à analyser.

La bande flexible de chaque articulation 6 flexible peut être ajustable de manière continue et par fléchissement, entre un angle de - 30 degrés (fléchissement selon un premier sens par rapport au plan principal PP) et un angle de + 30 degrés (fléchissement selon un second sens par rapport au plan principal PP), où ces maximums de fléchissement à - 30 et + 30 degrés correspondent à des limites de fléchissement propres à la bande flexible. Bien entendu, ces maximums de fléchissement à - 30 et + 30 degrés peuvent être plus élevés en valeur absolue. A titre d'exemple, avec une angulation totale de 30° entre deux supports 3, trois supports 3 alignés peuvent offrir un angle de 90° au capteur physiologique 1. Ces caractéristiques angulaires sont susceptibles d'être différentes selon la conception et les applications visées.

Dans le second mode de réalisation décrit ci-dessus où les articulations 6 sont des articulations semi-rigides, chaque articulation 6 semi-rigide peut être munis de crans de réglage de l'angle d'inclinaison entre les deux supports 3 selon plusieurs valeurs prédéfinies, pour un ajustage naturelle, lors de l'application sur la partie du corps du patient à analyser, de manière discrète par échelons ou pas d'angles d'inclinaison déterminés. Chaque articulation 6 semi-rigide forme ainsi une charnière crantée, où à chaque cran de réglage correspond un angle d'inclinaison.

Chaque articulation 6 semi-rigide peut être ajustable selon plusieurs angles d'inclinaison prédéfinis compris entre 0 degré et 30 degrés, avec une séparation d'un pas (ou échelon) angulaire constant, notamment un pas angulaire compris entre 5 et 15 degrés. Avec une angulation totale de 30° entre deux supports 3, trois supports 3 alignés peuvent offrir un angle de 90° au capteur physiologique 1. Ces caractéristiques angulaires sont susceptibles d'être différentes selon la conception et les applications visées.

Chaque articulation 6 semi-rigide peut par exemple présenter les angles d'inclinaison suivants, en degrés, [0, 5, 10, 15, 20, 25, 30] ou [0, 10, 20, 30] ou [0, 15, 30].

Chaque articulation 6 semi-rigide est également muni de deux butées pour bloquer l'inclinaison entre une valeur minimale et une valeur maximale, par exemple entre 0 degré et 30 degrés ou entre - 30 et + 30 degrés.

Afin de connaître automatiquement l'angle d'inclinaison, parmi les différentes valeurs discrètes offertes, chaque articulation 6 (qu'elle soit flexible ou semi-rigide) entre deux supports 3 est couplée à un capteur d'angle (non illustré sur les figures 1a à 3a et 8a, 8b, et illustré par contre sur les figures 9a, 9b et 10) configuré pour la mesure de l'angle d'inclinaison entre les deux supports 3.

Dans le premier mode de réalisation, le capteur d'angle se présente sous la forme d'un capteur résistif flexible délivrant une donnée de mesure traduisant une variation de résistance en fonction du fléchissement de la bande flexible. Un tel capteur résistif flexible, ou capteur de flexion, permet de mesurer l'angle d'inclinaison entre les deux supports 3 (qui correspond à l'angle de fléchissement de la bande flexible) en se basant sur l'exploitation des variations de résistance se produisant lorsqu'un matériau conducteur est courbé. Le capteur résistif flexible se comporte ainsi comme un potentiomètre passif dont la résistance varie en fonction du fléchissement.

Un tel capteur résistif flexible peut :
- soit être intégré à la bande flexible,
- soit être intégré à une lame flexible fixée à la bande flexible, notamment de manière détachable (par exemple par collage, adhésif, aimantation, clipsage, pressage, ...) de sorte qu'un fléchissement de la bande flexible conduit à un fléchissement de la lame flexible, et donc à un fléchissement du matériau conducteur du capteur résistif flexible.

Dans le cadre du premier mode de réalisation avec la membrane flexible 300, deux réalisations distinctes sont envisageable pour l'intégration de capteurs résistifs 60.

Dans la réalisation des figures 9a et 9b, les capteurs résistifs 60 flexibles sont intégrés à la membrane flexible 300, en étant par exemple au moins partiellement noyés dans une couche en polymère élastomérique.

De tels capteurs résistifs 60 peuvent être disposés entre les ilôts délimitant les modules élémentaires 2.

En variante, pour des raisons d'encombrement notamment, et comme visible sur les figures 9a et 9b, les capteurs résistifs 60 peuvent être déportés sur les côtés de la membrane flexible 300, en disposant des capteurs résistifs 60 le long des axes d'articulations AX, AY, de sorte que les capteurs résistifs 60 pourront mesurer des fléchissements au niveau des bandes flexibles le long des axes d'articulation correspondants. Dans l'exemple des figures 9a et 9b, les capteurs résistifs 60 qui mesurent des fléchissement selon des axes d'artivculation AY, parallèle à la direction Y, sont répartis de manière alternée entre les deux côtés longitudinaux (côtés selon la direction X) de la membrane flexible 300. Il est donc à noter qu'un capteur résistif 60 peut être couplé à deux ou plus articulations.

Dans la réalisation de la figure 10, les capteurs résistifs 60 flexibles sont intégrés à un film flexible 600 qui est rapporté et fixé (notamment de manière amovible) sur la membrane flexible 300, avec les capteurs résistifs flexibles qui peuvent soit être superposés avec les bandes flexibles qui pour rappel correspondent aux parties de la membrane flexible situés entre les ilôts délimitant les modules élémentaires 2, soit être déportés sur les côtés du film flexible 600 selon la même conformation déportée que sur les figures 9a et 9b.

Si le film flexible 600 se positionne sur l'avant de la membrane flexible 300 et vient recouvrir les composants optoélectroniques 4, 5, alors ce film flexible 600 est nécessairement transparent. Par contre, si le film flexible 600 se positionne sur l'arrière de la membrane flexible 300, alors ce film flexible 600 peut être opaque ou transparent.

Ainsi, la membrane flexible 300 avec ses composants optoélectroniques 4, 5 peut être à usage unique ou non. De même, le film flexible 600 avec ses capteurs résistifs 60 flexibles peut être à usage unique ou être réutilisable indépendamment de la membrane flexible 300.

Dans le second mode de réalisation, le capteur d'angle est par exemple conformé pour détecter le cran de réglage occupé dans l'articulation 6 semi-rigide, ce cran de réglage pouvant alors être associé à un angle d'inclinaison. Ce capteur d'angle peut par exemple comprendre des contacteurs couplés à chaque cran de réglage.

A titre d'exemple non limitatif, chaque articulation 6 semi-rigide peut comprendre :
- plusieurs billes rétractables coopérant avec un élément élastique de poussée, tels qu'un ressort ou une lame souple ou élastique, où ces billes sont insérées dans un rail courbe d'une courbure correspondant au secteur d'angle d'inclinaison maximal souhaité et ce rail est monté coulissant à l'intérieur d'un guide de même courbure, et
- un curseur sur le guide apte à écraser les billes selon le coulissement du rail au sein du guide, où les supports 3 liés par l'articulation 6 sont respectivement fixés à une extrémité du rail et à une extrémité du guide ;
- des butées qui empêchent le rail et le guide de se désolidariser, tout en bloquant l'inclinaison entre une valeur minimale et une valeur maximale.

En variante du curseur, il est envisageable de prévoir des évidements formant des crans sur le rail, libérant ainsi la pression sur les billes.

Il est à noter que les articulations 6 peuvent éventuellement être les supports des câbles de liaison pour les émetteurs 4 et les récepteurs 5 et/ou siège de connexions électriques souples liant deux modules élémentaires 2.

De manière générale, pour chaque module élémentaire 2, le ou les composants optoélectroniques 4, 5 sont tous montés sur une face frontale du support 3 concerné, cette face frontale correspondant à la face qui est appliquée sur la partie du corps à analyser. Le support 3 présente ainsi une face frontale, ainsi qu'une face dorsale opposée à la face frontale, cette face dorsale étant ainsi opposée à la partie du corps à analyser.

Il est également envisageable que pour chaque module élémentaire 2, le ou les composants optoélectroniques 4, 5 soient recouverts d'une couche de matière transparente.

Dans le premier mode de réalisation des figures 1a et 1b, chaque module élémentaire 2 comprend, sur son support 3, quatre émetteurs 4 émettant à des longueurs distinctes dans le proche infrarouge entre 650 et 1000 nanomètres, ainsi que quatre récepteurs 5.

Les quatre émetteurs 4 sont placés au centre du support 3 et les quatre récepteurs 5 sont placés en périphérie du support 3, autour des quatre récepteurs. Dans les calculs de soustraction spatiale, et de manière générale dans le traitement tridimensionnel des données issues des récepteurs 5, les quatre émetteurs 4 ainsi rassemblés pourront éventuellement être associés à une coordonnée spatiale commune.

Deux récepteurs 5 sont disposés de part et d'autre des émetteurs 4 selon une direction longitudinale parallèle à l'axe longitudinal X, de sorte que ces deux récepteurs 5 sont décalés axialement selon cette direction longitudinale, contribuant à accroître la résolution spectroscopique en longitudinal en multipliant les récepteurs 5.

De même, deux autres récepteurs 5 sont disposés de part et d'autre des émetteurs 4 selon une direction transversale parallèle à l'axe transversal Y, de sorte que ces deux autres récepteurs 5 sont décalés axialement selon cette direction transversale, contribuant aussi à accroître la résolution spectroscopique en transversal en multipliant les récepteurs 5.

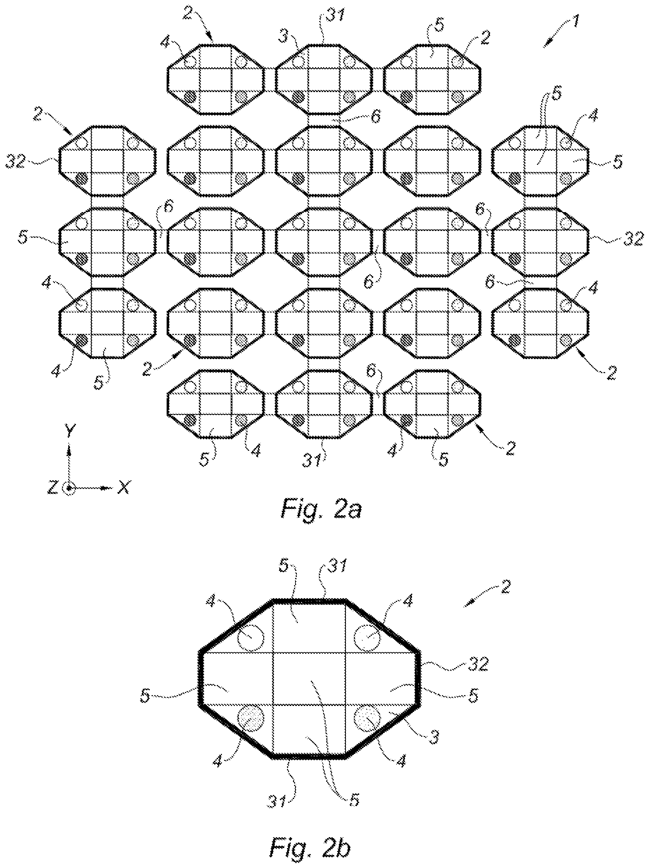

Dans le deuxième mode de réalisation des figures 2a et 2b, chaque module élémentaire 2 comprend, sur son support 3, quatre émetteurs 4 émettant à des longueurs distinctes dans le proche infrarouge entre 650 et 1000 nanomètres, ainsi que cinq récepteurs 5.

Un récepteur 5, dit récepteur central, est placé au centre du support 3 et les quatre autres récepteurs 5 sont placés en périphérie du support 3, autour du récepteur 5 central.

Deux récepteurs 5 sont disposés de part et d'autre du récepteur 5 central selon une direction longitudinale parallèle à l'axe longitudinal X, de sorte que ces deux récepteurs 5 et le récepteur 5 central sont décalés axialement selon cette direction longitudinale, contribuant à accroître la résolution spectroscopique en longitudinal en multipliant les récepteurs 5.

De même, deux autres récepteurs 5 sont disposés de part et d'autre du récepteur 5 central selon une direction transversale parallèle à l'axe transversal Y, de sorte que ces deux autres récepteurs 5 et le récepteur 5 central sont décalés axialement selon cette direction transversale, contribuant aussi à accroître la résolution spectroscopique en transversal en multipliant les récepteurs 5.

Les quatre émetteurs 4 sont placés aux quatre coins du support 3 entre les quatre récepteurs 5 disposés en périphérie.

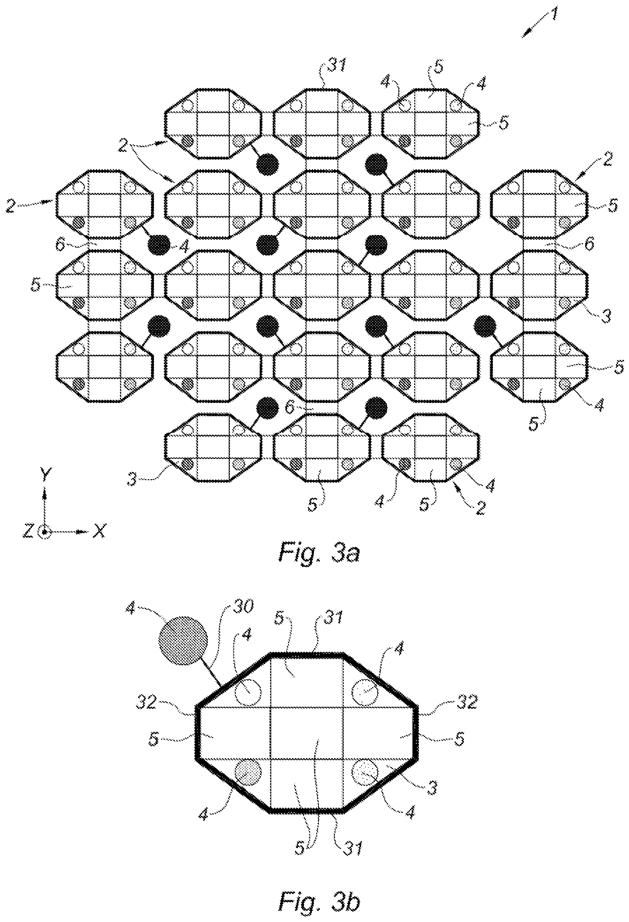

Dans le troisième mode de réalisation des figures 3a et 3b, chaque module élémentaire 2 est identique au module élémentaire 2 du deuxième mode de réalisation, à la différence que certains des modules élémentaires 2 supportent un autre émetteur 4 (voire éventuellement un autre récepteur) qui est porté par le support 3 via un bras 30 de manière à ce que ledit autre émetteur 4 (ou récepteur) soit disposé dans une zone interstitielle entre quatre modules élémentaires 2 voisins.

Cette solution avec émetteur 4 (ou récepteur) maintenu par un bras aurait aussi pu être envisagé avec le module élémentaire 2 du premier mode de réalisation.

Il aurait pu aussi être envisagé de prévoir sur certains modules élémentaires 2 uniquement un ou plusieurs émetteurs 4, et de prévoir sur les autres modules élémentaires 2 uniquement un ou plusieurs récepteurs 5.

Selon les tailles souhaitées des émetteurs 4 et des récepteurs 5, qui forment des composants optoélectroniques, et selon les applications visées, il est envisageable d'avoir plusieurs solutions :
- une combinaison uniforme de composants optoélectroniques (émetteurs et récepteurs) homogènes comme hétérogènes, formant un sous-ensemble ;
- de simples composants optoélectroniques unitaires, émetteurs et récepteurs ;
- une combinaison mixte des deux précédentes solutions.

Les émetteurs 4 peuvent être issus du commerce, peuvent être des fibres optiques couplés à une source en infrouge, des diodes lasers, des diodes électroluminescentes ou encore des composants optoélectroniques fabriqués en salle blanche. Les émetteurs 4 peuvent par exemple être constitués de diodes de tailles différentes, combinant diodes de grande et de petite taille.

Les récepteurs 5 peuvent être des capteurs CMOS, des photodiodes, des photorésistances, des phototransistors, des cellules photoélectriques ou encore des composants optoélectroniques fabriqués en salle blanche.

Dans le cas de modules élémentaires 2 comprenant au moins un émetteur 4, il est envisageable d'avoir plusieurs émetteurs 4 présentant des longueurs d'ondes différentes. Ces émetteurs 4 sont alors avantageusement regroupées, notamment au centre du support 3, en vue d'être considérés lors du traitement comme une unique source de lumière. Ainsi, ces émetteurs 4 se comporte comme une source de lumière unique à longueur d'ondes multiples. Le choix et le nombre des longueurs d'ondes est fonction des applications visées, et notamment des métabolites et des molécules ciblés.

De par sa capacité à se conformer à la morphologie d'un organe ou d'une partie du corps, le capteur physiologique 1 permet notamment la surveillance de la perfusion d'un éventail large d'organes ou de parties de corps.

Ainsi, un tel capteur physiologique 1 peut être envisagé, de par sa conformabilité, pour surveiller la perfusion tissulaire de zones sous-cutanées variées, à commencer par le cortex frontal, mais aussi les reins, le mésentère et les muscles. Ce capteur physiologique 1 peut également être envisagé, de par sa conformabilité et dans des conditions stériles, pour surveiller la perfusion d'organes durant une intervention chirurgicale, tels que le muscle myocardique au cour d'une chirurgie cardiaque.

Les articulations 6 de ce capteur physiologique 1 permettent en effet de contourner les contraintes morphologiques de la surface d'application ou de mesure, dont la création de couches d'air diffusantes. Ces articulations 6 permettent également de réduire la longueur des chemins optiques à l'intérieur des tissus biologiques, tout en gagnant en profondeur de mesure, en utilisant le relief et la rotondité parfois de leur forme. Dans une configuration particulière, le capteur physiologique 1 peut être configuré et positionné en demi arc de cercle de sorte que des composants optoélectroniques 4, 5 peuvent se retrouver alignés en vis-à-vis de part et d'autre de l'organe ou de la partie du corps.

Dans le cadre d'un système de spectroscopie en proche infrarouge, un tel capteur physiologique 1 est employé en association avec une unité de contrôle (non illustrée) comme un terminal informatique, un contrôleur, un microcontrôlleur, un processeur, un microprocesseur, une carte électronique, carte d'acquisition et de traitement de données, ou tout au autre assemblage de composants électroniques adapté pour la mise en oeuvre des fonctionnalités évoquées ci-après.

L'unité de contrôle est ainsi reliée, par voie filaire ou non filaire, aux émetteurs 4, aux récepteurs 5 et aux capteurs d'angle des articulations 6. Chaque émetteur 4 et chaque récepteur 5 est adressé individuellement par l'unité de contrôle.

Dans le cas d'une voie filaire, une nappe de câbles relie l'unité de contrôle aux émetteurs 4, récepteurs 5 et aux capteurs d'angle, sans gêner la mobilité du capteur physiologique 1. Le câblage est de préférence situé au dos des modules élémentaires 2 (c'est-à-dire sur la face dorsale du support 3) afin de ne pas réduire la surface utile du capteur physiologique 1 (c'est-à-dire la surface des faces frontales des supports 3).

Dans le cas d'une voie non filaire, des câbles relient les émetteurs 4, récepteurs 5 et capteurs d'angle à un système de radiocommunication porté par le capteur physiologique 1, par exemple au dos d'un module élémentaire 2 (autrement dit sur une face dorsale d'un support 3) ou bien sur un support 3 dédié qui ne porte pas nécessairement d'émetteur 4 et de récepteur 5. Ce système de radiocommunication est en radiocommunication bidirectionnelle avec l'unité de contrôle pour envoyer et recevoir des données.

Le système de radiocommunication peut par exemple comprendre une puce de radiocommunication courte portée (par exemple une portée maximale comprise entre un et cinq mètres, voire une quinzaine de mètres) selon une technologie WBAN «Wireless Body Area Network» ou WBASN «Wireless Body Area Sensor Network», afin de pouvoir établir une radiocommunication sur un réseau WBAN ou WBASN entre le capteur physiologique 1 et l'unité de contrôle. A titre d'exemple non limitatif, parmi les différents protocoles de technologie WBAN ou WBASN, peuvent être retenus les protocoles Zigbee^{™} et Bluetooth^{™} Low Energy (BLE).

L'unité de contrôle est configurée pour remplir plusieurs fonctionnalités dont principalement les quatre fonctionnalités décrites ci-après, autrement dit pour réaliser plusieurs étapes.

Une première fonctionnalité de l'unité de contrôle (ou une première étape) est d'établir les coordonnées spatiales de chaque émetteur 4 et de chaque récepteur 5 en fonction :
- des angles d'inclinaison des articulations 6 ;
- du positionnement du module élémentaire 2 correspondant dans le capteur physiologique 1 (par exemple 3^{ème} ligne et 1^{ère} colonne) ;
- du positionnement de l'émetteur 4 ou récepteur 5 au sein même du module élémentaire 2 correspondant.

Ces coordonnées spatiales sont établies une fois le capteur physiologique 1 appliqué sur la partie du corps du patient à analyser, et donc une fois que le capteur physiologique 1 est dans la conformation incurvée adéquate. Le capteur d'angle de chaque articulation 6 communique, à l'unité de contrôle, la valeur de l'angle d'inclinaison ou l'indice du cran de réglage ou la valeur de la résistance du potentiomètre passif (selon la technologie retenue) que l'unité de contrôle traduira en angle d'inclinaison.

Cette première fonctionnalité peut être répétée de manière itérative ou régulière, afin de connaître de manière dynamique ces coordonnées spatiales. En effet, la partie du corps du patient à analyser peut évoluer dans sa forme et dans sa taille, comme par exemple gonfler ou dégonfler, et dans ce cas la conformation incurvée du capteur physiologique 1 change et donc les coordonnées spatiales changent. Autrement dit, une commande de l'unité de contrôle actualise à chaque cycle (un cycle correspondant à la réalisation des quatres fonctionnalités ou étapes citées) l'information relative aux angles d'inclinaison. Ainsi, la représentation tridimensionnelle du capteur physiologique 1 est actualisée à chaque cycle de mesure au sein du repère orthonormé (X, Y, Z) du volume de mesure.

Ainsi, chaque émetteur 4 et chaque récepteur 5 sont localisés par leurs coordonnées dans un même repère orthonormé (X, Y, Z), dans le même espace virtuel en trois dimensions que celui du corps ou des tissus analysés.

L'unité de contrôle permet ainsi de construire une empreinte optoélectronique tridimensionnelle et virtuelle du capteur physiologique 1 en situation sur l'organe ou sur la partie du corps, et cette empreinte optoélectronique tridimensionnelle est placée dans le repère orthonormé (X, Y, Z), comme visible sur les figures 5a et 5b, pour servir à l'analyse des signaux mesurées par les récepteurs 5, et donc à la représentation graphique des volumes analysés sous le capteur physiologique 1, ainsi qu'expliqué ultérieurement.

La discrimination des différents plans biologiques sous le capteur physiologique 1, selon leur vascularisation, permet de repérer la zone à examiner. Cette zone à examiner peut être définie par son empreinte optoélectronique tridimensionnelle à la longueur d'onde de 800 nanomètres par exemple, afin de représenter le volume de mesure.

Une deuxième fonctionnalité (ou une deuxième étape) est d'activer les émetteurs 4 de manière individuelle et non concomitante selon une séquence prédéfinie. Autrement dit, l'unité de contrôle active les émetteurs 4 de façon alternative. Les émetteurs 4 peuvent être synchronisés entre eux pour une illumination en chenillard, les uns à la suite des autres, sans qu'à aucun moment deux émetteurs 4 ne soient activés en même temps.

Une troisième fonctionnalité (ou une troisième étape) est une récupération et un traitement tridimensionnel (par exemple selon une méthode de soustraction spatiale) des mesures issues des récepteurs 5 durant la séquence en fonction des coordonnées spatiales des émetteurs 4 et des récepteurs 5.

Une quatrième fonctionnalité (ou une quatrième étape) est de construire une image tridimensionnelle représentant un volume vasculaire ou sanguin dans un volume d'analyse, ce volume vasculaire ou sanguin étant composé de voxels VO construits à partir du traitement tridimensionnel, notamment selon une méthode de soustraction spatiale, des mesures issues des récepteurs durant la séquence.

L'acquisition des données est basée sur une modulation spatiale et temporelle des émissions en infrarouge. L'unité de contrôle est en mesure d'acquérir des images durant une même période, assurant de ce fait la cohérence temporelle de l'acquisition.

La cohérence temporelle entre les émetteurs 4 et l'acquisition par les récepteurs 5 fait l'objet d'une commande de synchronisation qui est directement intégrée au sein de l'unité de contrôle. En variante, chaque module élémentaire 2 intègre directement un module de synchronisation entre les émetteurs 4 et les récepteurs 5.

Pour la mise en oeuvre de la méthode de soustraction spatiale, chaque combinaison différente d'émetteur 4 et de récepteur 5 forme un couple de mesure 4, 5. Chaque couple de mesure 4, 5 est unique à la fois par :
- l'axe de mesure AM qui passe par l'émetteur 4 et par le récepteur 5, et qui est assimilé à l'axe du faisceau en proche infrarouge FI émis par l'émetteur 4, traversant les tissus de la partie du corps ou de l'organe, et reçu par le récepteur 5 ; et par
- l'espacement (ou écartement) entre l'émetteur 4 et le récepteur 5.

La valeur d'absorption mesurée au niveau d'un récepteur 5 qui découle de l'activation d'un émetteur 4 est une information unique sur la localisation d'hémoglobines dans les tissus traversés par le faisceau en proche infrarouge FI associé au couple de mesure 4, 5 concerné. La figure 4 illustre plusieurs couples de mesure 4, 5 au moyen de flèches qui représentent schématiquement des faisceaux en proche infrarouge de huit couples de mesure 4, 5 tous associés à un même émetteur 4, dont cinq couples de mesure 4, 5 suivant l'axe transversal Y et trois couples de mesure 4, 5 suivant l'axe longitudinal X.

La trajectoire du faisceau en proche infrarouge pour un couple de mesure 4, 5 est curviligne ou prend la forme d'un arc de lumière diffuse qui traverse les tissus organiques, comme schématisé sur les figures 5a et 5b, et sa profondeur relative PR par rapport à l'axe de mesure AM correspond à environ un tiers de l'espacement entre l'émetteur 4 et le récepteur 5 du couple de mesure.

Compte tenu de l'hétérogénéité vasculaire des tissus traversés, et donc d'une d'absorption lumineuse hétérogène, il est avantageux d'opérer une succession de soustractions spatiales afin de rendre plus sélective les mesures d'absorption, et le capteur physiologique 1 selon l'invention permet de répondre à ce point. En effet, chaque écartement émetteur 4/récepteur 5 étant connu, de même chaque écartement entre les récepteurs 5 étant également connu, toutes les combinaisons de couples de mesure 4, 5 ayant le même émetteur 4 sont répertoriés par l'unité de contrôle.

Pour une soustraction spatiale, chaque émetteur 4 est associé à au moins une paire de récepteurs 5, et de préférence plusieurs paires de récepteurs 5, pour une soustraction des mesures d'absorption entre les récepteurs 5 d'une même paire ; la ou les paires de récepteurs 5 considérés et associés à l'émetteur 4 présentent le même axe de mesure, autrement dit l'émetteur 4 et les récepteurs 5 sont alignés sur le même axe de mesure pour opérer la soustraction spatiale.

Les émetteurs 4 et les paires de récepteurs 5 qui leurs sont rattachées sont synchronisées dans un même temps d'illumination. L'ensemble de ces illuminations successives se réalisent à une vitesse propre à considérer qu'elles ont lieu dans une même unité de temps.

Ainsi, l'empreinte optoélectronique construite lors de la première étape (ou avec la première fonctionnalité) sert à établir les émetteurs 4 et récepteurs 5 appairés pour les soustractions spatiales, ces appairements étant tous singuliers et uniques de par leurs axes de mesure (singularité des chemins otiques) ains que les écartements et les angles d'inclinaison à considérer entre les composants optoélectroniques 4, 5 sollicités du capteur physiologique 1.

Chaque couple de mesure 4, 5 représente une valeur d'absorption en profondeur. Il est à noter que les coordonnées en X, Y et Z des composants optoélectroniques 4, 5 sont connues à partir de leurs dispositions sur le capteur physiologique 1 (disposition des modules élémentaires 2 au sein du capteur physiologique 1 et disposition des composants optoélectroniques 4, 5 sur leurs modules élémentaires 2 propres) et à partir des angles d'inclinaison concernés.

Cette valeur d'absorption en profondeur est ainsi associée à une profondeur relative PR vis-à-vis de l'axe de mesure AM du couple de mesure 4, 5, et qui correspond à un tiers de l'écartement entre l'émetteur 4 et le récepteur 5 concernés.

En se référant à la Figure 5b, si le capteur physiologique 1 présente une courbure entre l'émetteur 4 et le récepteur 5 du couple de mesure 4, 5 considéré, alors la profondeur relative PR est distincte de la profondeur de mesure PM, qui correspond à la profondeur de pénétration du faisceau en proche infrarouge FI dans la partie du corps ou de l'organe. Plus précisément, la profondeur de mesure PM correspond à l'addition de la profondeur relative PR et de la hauteur HA mesurée orthogonalement à l'axe de mesure AM entre :
- un point milieu MI situé sur l'axe de mesure AM, à mi distance entre l'émetteur 4 et le récepteur 5, et
- le capteur physiologique 1, autrement dit un point de contact CS entre le capteur physiologique 1 et la surface cutanée de la partie du corps ou de l'organe du patient.

En se référant à la Figure 5a, si le capteur physiologique 1 est plan entre l'émetteur 4 et le récepteur 5 du couple de mesure 4, 5 considéré, alors la profondeur relative PR correspond à la profondeur de mesure PM, autremetn dit la hauteur HA est nulle.

Lors de la troisième étape (ou avec la troisième fonctionnalité), la soustraction spatiale est réalisée entre tout ou partie des paires de couples de mesure 4, 5 de même émetteur 4 et de récepteurs 5 alignés selon le même axe de mesure AM, en soustrayant le signal le moins profond (celui correspondant au couple de mesure présentant le plus faible écartement entre l'émetteur 4 et le récepteur 5) au signal le plus profond (celui correspondant au couple de mesure présentant le plus grand écartement entre l'émetteur 4 et le récepeur 6).

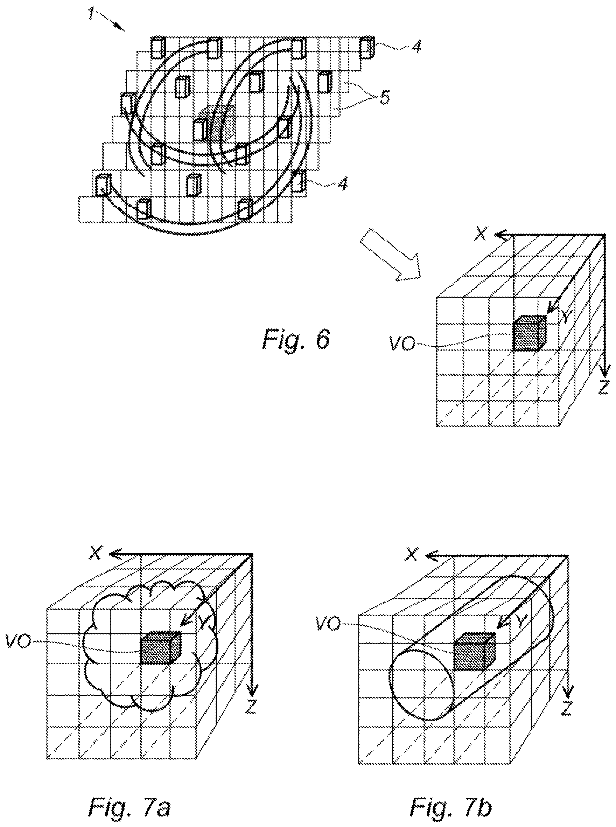

Lors de la quatrième étape (ou avec la quatrième fonctionnalité), le résultat de cette soustraction entre la valeur d'absorption du signal le moins profond et la valeur d'absorption du signal le plus profond est associée à des coordonnées en X, Y, Z et est ainsi consignée dans une carte volumétrique sous la forme d'un voxel VO, comme schématisé sur la figure 6, sur la figure 7a où est illustré un voxel VO issu d'une imagerie d'un volume sanguin de type hématome, ou sur la figure 7b où est illustré un voxel VO issu d'une imagerie d'un volume sanguin de type vaisseau sanguin.

Les coordonnées du voxel dépendent des coordonnées de l'émetteur 4 et des deux récepteurs 5 considérés, et dépendent donc, entre autres, de la ou des angles d'inclinaison concernés.

A titre d'exemple, si le capteur physiologique 1 est plan entre l'émetteur 4 et les récepteurs 5 considérés, l'axe de mesure qui passe par l'émetteur 4 et les récepteurs 5 alignés formera l'axe de référence. Si le capteur physiologique 1 présente une courbure entre l'émetteur 4 et le récepteur 5 du couple de mesure 4, 5 considéré, la bissectrice de l'angle formé par les axes passant par l'émetteur 4 et les deux récepteurs 5 formera l'axe de référence.

Lors de cette quatrième étape, des algorithmes de reconstruction, notamment basés sur des seuils de détection des photons, pourront servir à reconstituer une cartographie tridimensionnelle ainsi qu'une volumétrie de zones homogènes d'absorption. Les voxels peuvent être croisés entre eux, pour affiner la résolution.

Une variante à la méthode de soustraction spatiale pourrait être une méthode de tomographie par établissement d'une carte de contraste, ou encore une méthode de reconstruction à nuage de points consistant à considérer toutes les valeurs d'absorption par chaque récepteur 5 à chaque longueur d'onde pour former un nuage de points. Des algorithmes de reconstruction s'attacheront ensuite à en identifier la cohérence en lien avec les différents volumes d'absorption homogène, afin d'en permettre une représentation graphique tridimensionnelle.

De façon principale et non limitative, le capteur physiologique 1 est privilégié pour les applications médicales intéressant l'hémodynamique des patients, en mettant en oeuvre des longueurs d'ondes affines avec les hémoglobines telle que 803 nanomètres, exemple de point isobestic d'absorption des hémoglobines, ou encore les longueurs d'ondes davantage affines avec les hémoglobines oxygénée et désoxygénées.

Une longueur d'onde proche du point isobestic des hémoglobines à 803 nanomètres peut ainsi être utilisée pour établir une carte tridimensionnelle à fort contraste. Les hémoglobines ne circulant que dans le réseau vasculaire, et les organes, os et téguments étant de vascularisation variable, une discrimination des tissus biologiques peut ainsi être opérée. Par exemple, dans le cas de la surveillance du cortex frontal, le capteur physiologique 1 permet de discriminer le scalp, la boîte crânienne, la substance blanche de la substance grise, chacune de densité vasculaire différente.

D'autres longueurs d'ondes dans le proche infrarouge, de part et d'autre de 800 nanomètres peuvent être utilisées pour la discriminations des hémoglobines oxygénées des hémoglobines désoxygénées. Les chemins optiques des faisceaux lumineux de ces différentes longueurs d'ondes peuvent alors soit être considérés comme similaires à ceux de la longueur d'onde à 800 nanomètres, en raison de leur juxtaposition à celle-ci sur les modules élémentaires 2 concernés, soit être considérés relativement à ceux-ci au cours du traitement par les algorithmes de reconstruction. Le processus de représentation tridimensionnelle des modules élémentaires 2 et des traitements tridimensionnelles (eg. selon une méthode de soustraction spatiale, une méthode de tomographie, ou selon une méthode de reconstruction à nuage de points) est appliqué à l'identique du procédé avec les quatre étapes précédemment décrites.

Une première application est le dosage local ou régional, dynamique, en tendance ou en valeur absolue, du tissu en molécule ciblée. Ce dosage a pour objet de connaître la concentration, c'est-à-dire le rapport entre la quantité de la molécule ciblée sur le volume de mesure. Les cartes tridimensionnelles obtenues aux différentes longueurs d'ondes traduisant des niveaux d'absorption tissulaire variables sont combinées et superposées, pour pouvoir extraire le dosage.

Pour la discrimination des hémoglobines oxygénées et désoxygénées au sein du volume de mesure, et le suivi dynamique, en tendance ou en valeur absolue de leur proportion au sein de ce volume de mesure, les chemins optiques sont reproduits à au moins une autre longueur d'ondes spécifique affine avec la désoxyhémoglobine (Hb), par exemple entre 650 et 800 nanomètres. Au moins une seconde carte tridimensionnelle obtenue avec cette autre longueur d'onde différente permettra, par combinaison à la première carte tridimensionnelle obtenue avec la longueur d'onde proche de 800 nanomètres, de représenter les niveaux d'oxygénation des diverses strates tissulaires identifiées. La précision de la discrimination des molécules recherchées peut être accrue par l'augmentation du nombre de longueur d'ondes utilisées. La quantification de leur absorption rapportée à un volume de mesure cible (par exemple volume de cortex frontal et temporal) permet leur dosage local et le calcul d'une valeur, relative ou absolue, de la saturation tissulaire en oxygène (STO2). D'autre part, toujours en lien avec ce volume tissulaire connu, la quantité régionale d'hémoglobine pourra y être calculée, de même que sa concentration.

Pour le dosage des métabolites, autres que les hémoglobines, les chemins optiques sont reproduits aux longueurs d'ondes spécifiques au métabolite ciblé, permettant de le discriminer, et ainsi d'obtenir un dosage in vivo de ce métabolite, régional, à l'échelle de l'organe.

Une deuxième application est la détermination du volume vasculaire cérébral et du plateau d'autorégulation. Les hémoglobines ne circulant que dans le réseau vasculaire, à hématocrite constante, l'invention permet une mesure continue des variations d'absorption à 800 nm, et donc une mesure des variations dynamiques du réseau vasculaire (vasodilatation et vasoconstriction) sous le capteur physiologique 1 qui épouse la la rotondité de la surface des hémisphères frontaux. Ainsi, avec le capteur physiologique 1, il est possible d'obtenir une mesure ponctuelle, voire continue, et non invasive du volume vasculaire cérébral après calibration de l'hématocrite. Cette donnée physiologique pourra être combinée à d'autres permettant de déterminer le seuil d'autorégulation cérébral des patients.

Une troisième application est la mesure de la réserve de vasodilatation et des propriétés endothéliales.

Une quatrième application est l'évaluation de la perfusion tissulaire dans le cas des arthériopathie oblitérante des membres inférieurs.

## Revendications

1. Système de spectroscopie en proche infrarouge, comprenant un capteur physiologique (1) pour une spectroscopie en proche infrarouge à différentes profondeurs, ledit capteur physiologique (1) intégrant des composants optoélectroniques (4, 5) comprenant des émetteurs (4) de faisceaux en proche infrarouge et des récepteurs (5) photosensibles en proche infrarouge, ledit capteur physiologique (1) comprenant des modules élémentaires (2) pourvus chacun d'au moins un composant optoélectronique (4, 5), où chaque module élémentaire (2) est relié à au moins un module élémentaire (2) adjacent au moyen d'une articulation (6) configurée pour un mouvement relatif entre les modules élémentaires (2) selon plusieurs angles d'inclinaison, où les modules élémentaires (2) sont assemblés au moins au moyen des articulations (6) et forment une nappe optoélectronique configurable entre :
- une configuration plane selon un plan principal (PP) dans laquelle chaque articulation (6) présente un angle d'inclinaison nul vis-à-vis dudit plan principal (PP), et
- une multiplicité de conformations incurvées dans laquelle l'une au moins des articulations (6) présente un angle d'inclinaison non nul vis-à-vis dudit plan principal (PP) ;
ledit système de spectroscopie en proche infrarouge comprenant en outre une unité de contrôle reliée aux émetteurs (4) et aux récepteurs (5) du capteur physiologique (1) ;
ledit système de spectroscopie en proche infrarouge étant **caractérisé en ce que** chaque articulation (6) entre deux modules élémentaires (2) est couplée à un capteur d'angle configuré pour une mesure de l'angle d'inclinaison entre les deux modules élémentaires (2) ;
et **en ce que** ladite unité de contrôle est reliée à chaque capteur d'angle (60) et est configurée pour établir les coordonnées spatiales de chaque émetteur (4) et de chaque récepteur (5) en fonction des données de mesure issues desdits capteurs d'angle (60) et des positionnements et des écartements relatifs entre les émetteurs (4) et les récepteurs (5),
où ladite unité de contrôle est reliée, d'une part, aux émetteurs (4) du capteur physiologique (1) pour activer lesdits émetteurs (4) de manière individuelle et non concomitante selon une séquence prédéfinie et, d'autre part, aux récepteurs (5) du capteur physiologique (1) pour une récupération et un traitement tridimensionnel des mesures issues des récepteurs (5) durant ladite séquence en fonction des coordonnées spatiales des émetteurs (4) et des récepteurs (5).

2. Système de spectroscopie en proche infrarouge selon la revendication 1, dans lequel au moins une ou chaque articulation entre deux modules élémentaires (2) est une articulation flexible comprenant une bande flexible configurée pour un mouvement relatif entre les modules élémentaires (2) par fléchissement de la bande flexible.

3. Système de spectroscopie en proche infrarouge selon la revendication 2, dans lequel chaque module élémentaire (2) comprend un support (3), rigide ou souple, sur lequel est disposé au moins un composant optoélectronique (4, 5), et la bande flexible de l'articulation flexible (6) entre deux modules élémentaires (2) est reliée entre les supports (3) desdits deux modules élémentaires (2).

4. Système de spectroscopie en proche infrarouge selon la revendication 3, comprenant une membrane flexible (300) sur lequel les composants optoélectroniques (4, 5) sont disposés et répartis en ilôts délimitant les modules élémentaires (2), où la bande flexible de l'articulation flexible (6) entre deux modules élémentaires (2) est formée d'une partie de la membrane flexible (300) s'étendant entre les ilôts délimitant lesdits deux modules élémentaires (2).

5. Système de spectroscopie en proche infrarouge selon la revendication 4, dans lequel la membrane flexible (300) comprend au moins une couche en polymère élastomérique dans laquelle les composants optoélectroniques (4, 5) sont au moins partiellement noyés.

6. Système de spectroscopie en proche infrarouge selon l'une quelconque des revendications 2 à 5, dans lequel, pour une articulation flexible comprenant une bande flexible, ladite articulation flexible est couplée à un capteur d'angle comprenant un capteur résistif flexible (60) délivrant une donnée de mesure traduisant une variation de résistance en fonction du fléchissement de la bande flexible.

7. Système de spectroscopie en proche infrarouge selon la revendication 6, dans lequel le capteur résistif flexible (60) est intégré à la bande flexible ou est déporté par rapport à la bande flexible en étant intégré à une lame flexible fixée à la bande flexible, notamment de manière détachable, de sorte qu'un fléchissement de la bande flexible conduit à un fléchissement de la lame flexible.

8. Système de spectroscopie en proche infrarouge selon l'une quelconque des revendications précédentes, dans lequel chaque module élémentaire (2) comprend un support (3), rigide ou souple, sur lequel est disposé au moins un composant optoélectronique (4 ; 5), et chaque support (3) comprend deux bords longitudinaux (31) opposés et parallèles, et deux bords transversaux (32) opposés et parallèles, et chaque articulation (6) relie soit deux bords transversaux (32) contigus de deux supports (3) respectifs adjacents, soit deux bords longitudinaux (31) contigus de deux supports (3) respectifs adjacents.

9. Système de spectroscopie en proche infrarouge selon l'une quelconque des revendications précédentes, dans lequel, dans la configuration plane de la nappe optoélectronique, chaque articulation (6) entre deux modules élémentaires (2) est une articulation (6) pivotante autour d'un axe d'articulation s'étendant dans le plan principal (PP).

10. Système de spectroscopie selon l'une quelconque des revendications précédentes, dans lequel l'unité de contrôle est configurée pour un traitement tridimensionnel des mesures issues des récepteurs (5) durant la séquence selon une méthode de soustraction spatiale, ou selon une méthode de tomographie avec établissement de cartes de contrastes, ou selon une méthode de reconstruction à nuage de points.

11. Système de spectroscopie selon la revendication précédente, dans lequel l'unité de contrôle est configurée pour un traitement tridimensionnel, à chaque activation d'un émetteur (4), uniquement des mesures issues de récepteurs (5) sélectionnés en fonction d'une localisation relative vis-à-vis dudit émetteur (4) activé établie à partir de leurs coordonnées spatiales.

12. Procédé de spectroscopie en proche infrarouge mettant en oeuvre un système de spectroscopie selon l'une quelconque des revendications précédentes, ledit procédé de spectroscopie comprenant les étapes suivantes :
- collecte des données de mesure issues des capteurs d'angle (60) couplés à chaque articulation (6) ;
- établissement des coordonnées spatiales de chaque émetteur (4) et de chaque récepteur (5) en fonction des données de mesure issues des capteurs d'angle (60) ;
- activation des émetteurs (4) de manière individuelle et non concomitante selon une séquence prédéfinie ;
- récupération et traitement tridimensionnel des mesures issues des récepteurs (5) durant ladite séquence en fonction des coordonnées spatiales des émetteurs (4) et des récepteurs (5).

13. Procédé de spectroscopie selon la revendication 12, dans lequel le traitement tridimensionnel des mesures issues des récepteurs (5) durant la séquence met en oeuvre une méthode de soustraction spatiale, ou une une méthode de tomographie avec établissement de cartes de contrastes, ou une méthode de reconstruction à nuage de points.

14. Procédé de spectroscopie selon l'une quelconque des revendications 12 et 13, dans lequel l'étape de récupération et traitement est suivie d'une étape de construction d'une image tridimensionnelle représentant un volume vasculaire ou sanguin dans un volume d'analyse, ledit volume vasculaire ou sanguin étant composé de voxels construits à partir du traitement tridimensionnel des mesures issues des récepteurs (5) durant la séquence.

## Patentansprüche

1. Nahinfrarot-Spektroskopiesystem, das einen physiologischen Sensor (1) für eine Nahinfrarot-Spektroskopie in verschiedenen Tiefen umfasst, wobei der physiologische Sensor (1) optoelektronische Komponenten (4, 5) umfasst, die Strahlensender (4) im nahen Infrarotbereich und lichtempfindliche Empfänger (5) im nahen Infrarotbereich umfassen, wobei der physiologische Sensor (1) Elementarmodule (2) umfasst, die jeweils mit mindestens einer optoelektronischen Komponente (4, 5) versehen sind, wobei jedes Elementarmodul (2) mit mindestens einem benachbarten Elementarmodul (2) mittels eines Gelenks (6) verbunden ist, das für eine Relativbewegung zwischen den Elementarmodulen (2) entsprechend mehreren Neigungswinkeln konfiguriert ist, wobei die Elementarmodule (2) mindestens mittels der Gelenke (6) zusammengefügt sind und eine optoelektronische Ausbreitung bilden, die zwischen Folgendem konfigurierbar ist:
- einer planaren Konfiguration gemäß einer Hauptebene (PP), in der jedes Gelenk (6) einen Neigungswinkel von Null in Bezug auf die Hauptebene (PP) aufweist, und
- eine Reihe gekrümmter Gestaltungen, bei denen mindestens eines der Gelenke (6) einen Neigungswinkel ungleich Null in Bezug auf die Hauptebene (PP) aufweist;
wobei das Nahinfrarot-Spektroskopiesystem ferner eine Steuereinheit umfasst, die mit den Sendern (4) und Empfängern (5) des physiologischen Sensors (1) verbunden sind;
wobei das Nahinfrarot-Spektroskopiesystem **dadurch gekennzeichnet ist, dass** jedes Gelenk (6) zwischen zwei Elementarmodulen (2) mit einem Winkelsensor gekoppelt ist, der für eine Messung des Neigungswinkels zwischen den beiden Elementarmodulen (2) konfiguriert ist;
und dadurch, dass die Steuereinheit mit jedem Winkelsensor (60) verbunden und dazu konfiguriert ist, die räumlichen Koordinaten jedes Senders (4) und jedes Empfängers (5) basierend auf den von den Winkelsensoren (60) ausgegebenen Messdaten und den Positionierungen und den relativen Abständen zwischen den Sendern (4) und den Empfängern (5) festzulegen, wobei die Steuereinheit einerseits mit den Sendern (4) des physiologischen Sensors (1), um die Sender (4) einzeln und nicht gleichzeitig gemäß einer vordefinierten Reihenfolge zu aktivieren, und andererseits mit den Empfängern (5) des physiologischen Sensors (1) für ein Abrufen und ein dreidimensionales Verarbeiten der von den Empfängern (5) während der Reihenfolge ausgegebenen Messungen basierend auf den räumlichen Koordinaten der Sender (4) und der Empfänger (5) verbunden ist.

2. Nahinfrarot-Spektroskopiesystem nach Anspruch 1, wobei mindestens ein oder jedes Gelenk zwischen zwei Elementarmodulen (2) ein flexibles Gelenk ist, das einen flexiblen Streifen umfasst, der für eine Relativbewegung zwischen den Elementarmodulen (2) durch Biegen des flexiblen Streifens konfiguriert ist.

3. Nahinfrarot-Spektroskopiesystem nach Anspruch 2, wobei jedes Elementarmodul (2) einen starren oder flexiblen Träger (3) umfasst, auf dem mindestens eine optoelektronische Komponente (4, 5) angeordnet ist, und der flexible Streifen des flexiblen Gelenks (6) zwischen zwei Elementarmodulen (2) zwischen den Trägern (3) der beiden Elementarmodule (2) verbunden ist.

4. Nahinfrarot-Spektroskopiesystem nach Anspruch 3, umfassend eine flexible Membran (300), auf der die optoelektronischen Komponenten (4, 5) angeordnet und in die Elementarmodule (2) begrenzenden Inseln verteilt sind, wobei der flexible Streifen des flexiblen Gelenks (6) zwischen zwei Elementarmodulen (2) durch einen Abschnitt der flexiblen Membran (300) gebildet wird, der sich zwischen den die beiden Elementarmodule (2) begrenzenden Inseln erstreckt.

5. Nahinfrarot-Spektroskopiesystem nach Anspruch 4, wobei die flexible Membran (300) mindestens eine elastomere Polymerschicht umfasst, in der die optoelektronischen Komponenten (4, 5) mindestens teilweise eingebettet sind.

6. Nahinfrarot-Spektroskopiesystem nach einem der Ansprüche 2 bis 5, wobei für ein flexibles Gelenk, das den flexiblen Streifen umfasst, das flexible Gelenk mit einem Winkelsensor gekoppelt ist, der einen flexiblen Widerstandssensor (60) umfasst, der einen Messwert liefert, der eine Widerstandsänderung basierend auf der Biegung des flexiblen Streifens widerspiegelt.

7. Nahinfrarot-Spektroskopiesystem nach Anspruch 6, wobei der flexible Widerstandssensor (60) in den flexiblen Streifen integriert oder in Bezug auf den flexiblen Streifen versetzt ist, indem er in eine am flexiblen Streifen befestigte flexible Lamelle, insbesondere lösbar, integriert ist, so dass eine Durchbiegung des flexiblen Streifens zu einer Durchbiegung der flexiblen Lamelle führt.

8. Nahinfrarot-Spektroskopiesystem nach einem der vorhergehenden Ansprüche, wobei jedes Elementarmodul (2) einen starren oder flexiblen Träger (3) umfasst, auf dem mindestens eine optoelektronische Komponente (4; 5) angeordnet ist, und jeder Träger (3) zwei gegenüberliegende und parallele Längskanten (31) und zwei gegenüberliegende und parallele Querkanten (32) umfasst, und jedes Gelenk (6) entweder zwei Querkanten (32), die an zwei benachbarte jeweilige Träger (3) angrenzen, oder zwei Längskanten (31) verbindet, die an zwei benachbarte jeweilige Träger (3) angrenzen.

9. Nahinfrarot-Spektroskopiesystem nach einem der vorhergehenden Ansprüche, wobei in der planaren Konfiguration der optoelektronischen Ausbreitung jedes Gelenk (6) zwischen zwei Elementarmodulen (2) ein Gelenk (6) ist, das um eine Gelenkachse schwenkt, die sich in der Hauptebene (PP) erstreckt.

10. Spektroskopiesystem nach einem der vorhergehenden Ansprüche, wobei die Steuereinheit für eine dreidimensionale Verarbeitung der von den Empfängern (5) während der Reihenfolge ausgegebenen Messungen gemäß einem räumlichen Subtraktionsverfahren oder gemäß einem Tomographieverfahren mit Kontrastkartenerstellung oder gemäß einem Punktwolken-Rekonstruktionsverfahren konfiguriert ist.

11. Spektroskopiesystem nach dem vorhergehenden Anspruch, wobei die Steuereinheit für eine dreidimensionale Verarbeitung bei jeder Aktivierung eines Senders (4) nur von Messungen, die von Empfängern (5) ausgegeben werden, die basierend auf einer relativen Position in Bezug auf den festgelegten aktivierten Sender (4) aus ihren räumlichen Koordinaten ausgewählt wurden, konfiguriert ist.

12. Nahinfrarot-Spektroskopieverfahren, das durch ein Spektroskopiesystem nach einem der vorhergehenden Ansprüche durchgeführt wird, wobei das Spektroskopieverfahren die folgenden Schritte umfasst:
- Sammeln von Messdaten, die von den mit jedem Gelenk (6) gekoppelten Winkelsensoren (60) ausgegeben werden;
- Festlegen der räumlichen Koordinaten jedes Senders (4) und jedes Empfänger (5) basierend auf den Messdaten, die von den Winkelsensoren (60) ausgegeben werden;
- einzelnes und nicht gleichzeitiges Aktivieren der Sender (4) gemäß einer vordefinierten Reihenfolge;
- Abrufen und dreidimensionales Verarbeiten der Messungen, die während der Reihenfolge von den Empfängern (5) basierend auf den räumlichen Koordinaten der Sender (4) und der Empfänger (5) ausgegeben werden.

13. Spektroskopieverfahren nach Anspruch 12, wobei das dreidimensionale Verarbeiten der von den Empfängern (5) während der Reihenfolge ausgegebenen Messungen gemäß einem räumlichen Subtraktionsverfahren oder gemäß einem Tomographieverfahren mit Kontrastkartenerstellung oder gemäß einem Punktwolken-Rekonstruktionsverfahren durchgeführt wird.

14. Spektroskopieverfahren nach einem der Ansprüche 12 und 13, wobei auf den Schritt des Abrufens und Verarbeitens ein Schritt des Erstellens eines dreidimensionalen Bilds folgt, das ein Gefäß- oder Blutvolumen in einem Analysevolumen darstellt, wobei das Gefäß- oder Blutvolumen aus Voxeln besteht, die von der dreidimensionalen Verarbeiten der von den Empfänger (5) während der Reihenfolge ausgegebenen Messungen erstellt wurden.

## Claims

1. A near-infrared spectroscopy system, comprising a physiological sensor (1) for near-infrared spectroscopy at different depths, said physiological sensor (1) integrating optoelectronic components (4, 5) comprising emitters (4) of near-infrared beams and near-infrared photosensitive receivers (5), said physiological sensor (1) comprising elementary modules (2) each provided with at least one optoelectronic component (4, 5), wherein each elementary module (2) is connected to at least one adjacent elementary module (2) by means of an articulation (6) configured for a relative movement between the elementary modules (2) according to several angles of inclination, wherein the elementary modules (2) are assembled at least by means of the articulations (6) and form an optoelectronic spread configurable between:
- a planar configuration according to a main plane (PP) in which each articulation (6) has a zero angle of inclination with respect to said main plane (PP), and
- a set of curved conformations in which at least one of the articulations (6) has a non-zero angle of inclination with respect to said main plane (PP);
said near-infrared spectroscopy system further comprising a control unit connected to the emitters (4) and to the receivers (5) of the physiological sensor (1);
said near-infrared spectroscopy system being **characterized in that** each articulation (6) between two elementary modules (2) is coupled to an angle sensor configured for a measurement of the angle of inclination between the two elementary modules (2);
and **in that** said control unit is connected to each angle sensor (60) and is configured to establish the spatial coordinates of each emitter (4) and of each receiver (5) according to the measurement data originating from said angle sensors (60) and to the relative positionings and spacings between the emitters (4) and the receivers (5), wherein said control unit is connected, on the one hand, to the emitters (4) of the physiological sensor (1) to activate said emitters (4) in an individual and non-concomitant manner, according to a predefined sequence and, on the other hand, to the receivers (5) of the physiological sensor (1) for a recovery and a three-dimensional treatment of the measurements originating from the receivers (5) during said sequence according to the spatial coordinates of the emitters (4) and of the receivers (5).

2. The near-infrared spectroscopy system according to claim 1, wherein at least one or each articulation between two elementary modules (2) is a flexible articulation comprising a flexible strip configured for a relative movement between the elementary modules (2) by bending of the flexible strip.

3. The near-infrared spectroscopy system according to claim 2, wherein each elementary module (2) comprises a rigid or flexible support (3) on which at least one optoelectronic component (4, 5) is disposed, and the flexible strip of the flexible articulation (6) between two elementary modules (2) is connected between the supports (3) of said two elementary modules (2).

4. The near-infrared spectroscopy system according to claim 3, comprising a flexible membrane (300) on which the optoelectronic components (4, 5) are disposed and distributed into islets delimiting the elementary modules (2), wherein the flexible strip of the flexible articulation (6) between two elementary modules (2) is formed by a portion of the flexible membrane (300) extending between the islets delimiting said two elementary modules (2).

5. The near-infrared spectroscopy system according to claim 4, wherein the flexible membrane (300) comprises at least one elastomeric polymer layer in which the optoelectronic components (4, 5) are embedded at least partially.

6. The near-infrared spectroscopy system according to any one of claims 2 to 5, wherein, for a flexible articulation comprising a flexible strip, said flexible articulation is coupled to an angle sensor comprising a flexible resistive sensor (60) outputting an angle datum reflecting a variation of resistance as a function of the bending of the flexible strip.

7. The near-infrared spectroscopy system according to claim 6, wherein the flexible resistive sensor (60) is integrated to the flexible strip or is offset with respect to the flexible strip by being integrated to a flexible blade fastened to the flexible strip, in particular in a detachable manner, so that a bending of the flexible strip results in a bending of the flexible blade.

8. The near-infrared spectroscopy system according to any one of the preceding claims, wherein each elementary module (2) comprises a rigid or flexible support (3), on which at least one optoelectronic component (4; 5) is disposed, and each support (3) comprises two opposite and parallel longitudinal edges (31), and two opposite and parallel transverse edges (32), and each articulation (6) links two transverse edges (32) contiguous to two adjacent respective supports (3), namely two longitudinal edges (31) contiguous to two adjacent respective supports (3).

9. The near-infrared spectroscopy system according to any one of the preceding claims, wherein, in the planar configuration of the optoelectronic spread, each articulation (6) between two elementary modules (2) is an articulation (6) pivoting about an axis of articulation extending in the main plane (PP).

10. The spectroscopy system according to any one of the preceding claims, wherein the control unit is configured for a three-dimensional treatment of the measurements originating from the receivers (5) during the sequence according to a spatial subtraction method, or according to a tomography method with contrasts maps establishment, or according to a point cloud reconstruction method.

11. The spectroscopy system according to the preceding claim, wherein the control unit is configured for a three-dimensional treatment, at each activation of an emitter (4), of only measurements originating from receivers (5) selected according to a relative location with respect to said activated emitter (4) established from their spatial coordinates.

12. A near-infrared spectroscopy method implementing a spectroscopy system according to any one of the preceding claims, said spectroscopy method comprising the following steps:
- collection of the measurement data originating from the angle sensors (60) coupled to each articulation (6);
- establishment of the spatial coordinates of each emitter (4) and of each receiver (5) according to the measurement data originating from the angle sensors (60);
- activation of the emitters (4) in an individual and non-concomitant manner according to a predefined sequence;
- recovery and three-dimensional treatment of the measurements originating from the receivers (5) during said sequence according to the spatial coordinates of the emitters (4) and of the receivers (5).

13. The spectroscopy method according to claim 12, wherein the three-dimensional treatment of the measurements originating from the receivers (5) during the sequence implements a spatial subtraction method, or a tomography method with contrasts maps establishment, or a point cloud reconstruction method.

14. The spectroscopy method according to any one of claims 12 and 13, wherein the recovery and treatment step is followed by a step of constructing a three-dimensional image representing a vascular or blood volume within an analysis volume, said vascular or blood volume being composed of voxels constructed from the three-dimensional treatment of the measurements originating from the receivers (5) during the sequence.
